# EUROPEAN PATENT SPECIFICATION

(11) **EP 2 195 334 B1**
(45) Date of publication and mention of the grant of the patent: **31.07.2013**
(21) Application number: 08802147.2
(22) Date of filing: 13.09.2008
(51) Int. Cl.: C07K 14/435

(54) **LIGAND BINDING DOMAINS OF NUCLEAR RECEPTORS IN CONTROLLABLE FORM AND METHODS INVOLVING THE SAME**
LIGANDENBINDUNGSDOMÄNEN NUKLEÄRER REZEPTOREN IN STEUERBARER FORM UND VERFAHREN, AN DENEN DIESE BETEILIGT SIND
DOMAINES DE LIAISON DE LIGANDS DE RÉCEPTEURS NUCLÉAIRES SE PRÉSENTANT SOUS FORME CONTRÔLABLE ET PROCÉDÉS LES IMPLIQUANT

(30) Priority: 27.09.2007 EP 07291163
(43) Date of publication of application: 16.06.2010
(73) Proprietor: SANOFI, 75008 Paris (FR)
(72) Inventor: SCHWAHN, Uwe, 65926 Frankfurt am Main (DE); LANGER, Thomas, 65926 Frankfurt am Main (DE)
(74) Representative: Körner, Kathrin
(86) International application number: PCT/EP2008/007596
(87) International publication number: WO 2009/040003

(56) References cited:
- WO-A-03/000732
- WO-A-03/093312

### SEQUENCE LISTING

<110> sanofi-Aventis Deutschland GmbH
<120> Ligand binding domains of nuclear receptors in controllable form and methods involving the same
<130> 104SAN0704
<160> 6
<170> PatentIn version 3.4
<210> 1
   <211> 253
   <212> PRT
   <213> Homo sapiens
<400> 1
   Ala Glu Leu Glu His Leu Ala Gln Asn Ile Ser Lys Ser His Leu Glu
   1               5                   10                  15
   Thr Cys Gln Tyr Leu Arg Glu Glu Leu Gln Gln Ile Thr Trp Gln Thr
               20                  25                  30
   Phe Leu Gln Glu Glu Ile Glu Asn Tyr Gln Asn Lys Gln Arg Glu Val
           35                  40                  45
   Met Trp Gln Leu Cys Ala Ile Lys Ile Thr Glu Ala Ile Gln Tyr Val
       50                  55                  60
   Val Glu Phe Ala Lys Arg Ile Asp Gly Phe Met Glu Leu Cys Gln Asn
   65                  70                  75                  80
   Asp Gln Ile Val Leu Leu Lys Ala Gly Ser Leu Glu Val Val Phe Ile
                   85                  90                  95
   Arg Met Cys Arg Ala Phe Asp Ser Gln Asn Asn Thr Val Tyr Phe Asp
               100                 105                 110
   Gly Lys Tyr Ala Ser Pro Asp Val Phe Lys Ser Leu Gly Cys Glu Asp
           115                 120                 125
   Phe Ile Ser Phe Val Phe Glu Phe Gly Lys Ser Leu Cys Ser Met His
       130                 135                 140
   Leu Thr Glu Asp Glu Ile Ala Leu Phe Ser Ala Phe Val Leu Met Ser
   145                 150                 155                 160
   Ala Asp Arg Ser Trp Leu Gln Glu Lys Val Lys Ile Glu Lys Leu Gln
                   165                 170                 175
   Gln Lys Ile Gln Leu Ala Leu Gln His Val Leu Gln Lys Asn His Arg
               180                 185                 190
   Glu Asp Gly Ile Leu Thr Lys Leu Ile Cys Lys Val Ser Thr Leu Arg
           195                 200                 205
   Ala Leu Cys Gly Arg His Thr Glu Lys Leu Met Ala Phe Lys Ala Ile
       210                 215                 220
   Tyr Pro Asp Ile Val Arg Leu His Phe Pro Pro Leu Tyr Lys Glu Leu
   225                 230                 235                 240
   Phe Thr Ser Glu Phe Glu Pro Ala Met Gln Ile Asp Gly
                   245                 250
<210> 2
   <211> 271
   <212> PRT
   <213> Artificial
<220>
   <223> RORalpha domain with His-tag and Prescission cleavage site
<400> 2
   Met Gly Ser Ser His His His His His His Leu Glu Val Leu Phe Gln
   1               5                   10                  15
   Gly Pro Ala Glu Leu Glu His Leu Ala Gln Asn Ile Ser Lys Ser His
               20                  25                  30
   Leu Glu Thr Cys Gln Tyr Leu Arg Glu Glu Leu Gln Gln Ile Thr Trp
           35                  40                  45
   Gln Thr Phe Leu Gln Glu Glu Ile Glu Asn Tyr Gln Asn Lys Gln Arg
       50                  55                  60
   Glu Val Met Trp Gln Leu Cys Ala Ile Lys Ile Thr Glu Ala Ile Gln
   65                  70                  75                  80
   Tyr Val Val Glu Phe Ala Lys Arg Ile Asp Gly Phe Met Glu Leu Cys
                   85                  90                  95
   Gln Asn Asp Gln Ile Val Leu Leu Lys Ala Gly Ser Leu Glu Val Val
               100                 105                 110
   Phe Ile Arg Met Cys Arg Ala Phe Asp Ser Gln Asn Asn Thr Val Tyr
           115                 120                 125
   Phe Asp Gly Lys Tyr Ala Ser Pro Asp Val Phe Lys Ser Leu Gly Cys
       130                 135                 140
   Glu Asp Phe Ile Ser Phe Val Phe Glu Phe Gly Lys Ser Leu Cys Ser
   145                 150                 155                 160
   Met His Leu Thr Glu Asp Glu Ile Ala Leu Phe Ser Ala Phe Val Leu
                   165                 170                 175
   Met Ser Ala Asp Arg Ser Trp Leu Gln Glu Lys Val Lys Ile Glu Lys
   180 185 190
   Leu Gln Gln Lys Ile Gln Leu Ala Leu Gln His Val Leu Gln Lys Asn
   195 200 205
   His Arg Glu Asp Gly Ile Leu Thr Lys Leu Ile Cys Lys Val Ser Thr
   210 215 220
   Leu Arg Ala Leu Cys Gly Arg His Thr Glu Lys Leu Met Ala Phe Lys
   225 230 235 240
   Ala Ile Tyr Pro Asp Ile Val Arg Leu His Phe Pro Pro Leu Tyr Lys
   245 250 255
   Glu Leu Phe Thr Ser Glu Phe Glu Pro Ala Met Gln Ile Asp Gly
   260 265 270
<210> 3
   <211> 6
   <212> PRT
   <213> Artificial
<220>
   <223> His-tag
<400> 3
   His His His His His His
   1 5
<210> 4
   <211> 8
   <212> PRT
   <213> Artificial
<220>
   <223> Prescission cleavage site
<400> 4
   Leu Glu Val Leu Phe Gln Gly Pro
   1 5
<210> 5
   <211> 1020
   <212> DNA
   <213> Artificial
<220>
   <223> DNA coding for ROR alpha domain withe His-tag and Prescission cleavage site (coding strand)
<400> 5
   gataaccatc tcgcaaataa ataagtattt tactgttttc gtaacagttt tgtaataaaa      60
   aaacctataa atattccgga ttattcatac cgtcccacca tcgggcgcgg atccatggga     120
   agtagccatc atcatcatca tcatctggaa gttctgttcc aggggcccgc agaattagaa     180
   caccttgcac agaatatatc taaatcgcat ctggaaacct gccaatactt gagagaagag     240
   ctccagcaga taacgtggca gaccttttta caggaagaaa ttgagaacta tcaaaacaag     300
   cagcgggagg tgatgtggca attgtgtgcc atcaaaatta cagaagctat acagtatgtg     360
   gtggagtttg ccaaacgcat tgatggattt atggaactgt gtcaaaatga tcaaattgtg     420
   cttctaaaag caggttctct agaggtggtg tttatcagaa tgtgccgtgc ctttgactct     480
   cagaacaaca ccgtgtactt tgatgggaag tatgccagcc ccgacgtctt caaatcctta     540
   ggttgtgaag actttattag ctttgtgttt gaatttggaa agagtttatg ttctatgcac     600
   ctgactgaag atgaaattgc attattttct gcatttgtac tgatgtcagc agatcgctca     660
   tggctgcaag aaaaggtaaa aattgaaaaa ctgcaacaga aaattcagct agctcttcaa     720
   cacgtcctac agaagaatca ccgagaagat ggaatactaa caaagttaat atgcaaggtg     780
   tctacattaa gagccttatg tggacgacat acagaaaagc taatggcatt taaagcaata     840
   tacccagaca ttgtgcgact tcattttcct ccattataca aggagttgtt cacttcagaa     900
   tttgagccag caatgcaaat tgatgggtaa gaattccgga gcggccgctg cagatctgat     960
   cctttcctgg gacccggcaa gaaccaaaaa ctcactctct tcaaggaaat ccgtaatgtt    1020
<210> 6
   <211> 10435
   <212> DNA
   <213> Artificial
<220>
   <223> vector
<400> 6
   aagctttact cgtaaagcga gttgaaggat catatttagt tgcgtttatg agataagatt      60
   gaaagcacgt gtaaaatgtt tcccgcgcgt tggcacaact atttacaatg cggccaagtt     120
   ataaaagatt ctaatctgat atgttttaaa acacctttgc ggcccgagtt gtttgcgtac     180
   gtgactagcg aagaagatgt gtggaccgca gaacagatag taaaacaaaa ccctagtatt     240
   ggagcaataa tcgatttaac caacacgtct aaatattatg atggtgtgca ttttttgcgg     300
   gcgggcctgt tatacaaaaa aattcaagta cctggccaga ctttgccgcc tgaaagcata     360
   gttcaagaat ttattgacac ggtaaaagaa tttacagaaa agtgtcccgg catgttggtg     420
   ggcgtgcact gcacacacgg tattaatcgc accggttaca tggtgtgcag atatttaatg     480
   cacaccctgg gtattgcgcc gcaggaagcc atagatagat tcgaaaaagc cagaggtcac     540
   aaaattgaaa gacaaaatta cgttcaagat ttattaattt aattaatatt atttgcattc     600
   tttaacaaat actttatcct attttcaaat tgttgcgctt cttccagcga accaaaacta     660
   tgcttcgctt gctccgttta gcttgtagcc gatcagtggc gttgttccaa tcgacggtag     720
   gattaggccg gatattctcc accacaatgt tggcaacgtt gatgttacgt ttatgctttt     780
   ggttttccac gtacgtcttt tggccggtaa tagccgtaaa cgtagtgccg tcgcgcgtca     840
   cgcacaacac cggatgtttg cgcttgtccg cggggtattg aaccgcgcga tccgacaaat 900
   ccaccacttt ggcaactaaa tcggtgacct gcgcgtcttt tttctgcatt atttcgtctt 960
   tcttttgcat ggtttcctgg aagccggtgt acatgcggtt tagatcagtc atgacgcgcg 1020
   tgacctgcaa atctttggcc tcgatctgct tgtccttgat ggcaacgatg cgttcaataa 1080
   actcttgttt tttaacaagt tcctcggttt tttgcgccac caccgcttgc agcgcgtttg 1140
   tgtgctcggt gaatgtcgca atcagcttag tcaccaactg tttgctctcc tcctcccgtt 1200
   gtttgatcgc gggatcgtac ttgccggtgc agagcacttg aggaattact tcttctaaaa 1260
   gccattcttg taattctatg gcgtaaggca atttggactt cataatcagc tgaatcacgc 1320
   cggatttagt aatgagcact gtatgcggct gcaaatacag cgggtcgccc cttttcacga 1380
   cgctgttaga ggtagggccc ccattttgga tggtctgctc aaataacgat ttgtatttat 1440
   tgtctacatg aacacgtata gctttatcac aaactgtata ttttaaactg ttagcgacgt 1500
   ccttggccac gaaccggacc tgttggtcgc gctctagcac gtaccgcagg ttgaacgtat 1560
   cttctccaaa tttaaattct ccaattttaa cgcgagccat tttgatacac gtgtgtcgat 1620
   tttgcaacaa ctattgtttt ttaacgcaaa ctaaacttat tgtggtaagc aataattaaa 1680
   tatgggggaa catgcgccgc tacaacactc gtcgttatga acgcagacgg cgccggtctc 1740
   ggcgcaagcg gctaaaacgt gttgcgcgtt caacgcggca aacatcgcaa aagccaatag 1800
   tacagttttg atttgcatat taacggcgat tttttaaatt atcttattta ataaatagtt 1860
   atgacgccta caactccccg cccgcgttga ctcgctgcac ctcgagcagt tcgttgacgc 1920
   cttcctccgt gtggccgaac acgtcgagcg ggtggtcgat gaccagcggc gtgccgcacg 1980
   cgacgcacaa gtatctgtac accgaatgat cgtcgggcga aggcacgtcg gcctccaagt 2040
   ggcaatattg gcaaattcga aaatatatac agttgggttg tttgcgcata tctatcgtgg 2100
   cgttgggcat gtacgtccga acgttgattt gcatgcaagc cgaaattaaa tcattgcgat 2160
   tagtgcgatt aaaacgttgt acatcctcgc ttttaatcat gccgtcgatt aaatcgcgca 2220
   atcgagtcaa gtgatcaaag tgtggaataa tgttttcttt gtattcccga gtcaagcgca 2280
   gcgcgtattt taacaaacta gccatcttgt aagttagttt catttaatgc aactttatcc 2340
   aataatatat tatgtatcgc acgtcaagaa ttaacaatgc gcccgttgtc gcatctcaac 2400
   acgactatga tagagatcaa ataaagcgcg aattaaatag cttgcgacgc aacgtgcacg 2460
   atctgtgcac gcgttccggc acgagctttg attgtaataa gtttttacga agcgatgaca 2520
   tgacccccgt agtgacaacg atcacgccca aaagaactgc cgactacaaa attaccgagt 2580
   atgtcggtga cgttaaaact attaagccat ccaatcgacc gttagtcgaa tcaggaccgc 2640
   tggtgcgaga agccgcgaag tatggcgaat gcatcgtata acgtgtggag tccgctcatt 2700
   agagcgtcat gtttagacaa gaaagctaca tatttaattg atcccgatga ttttattgat 2760
   aaattgaccc taactccata cacggtattc tacaatggcg gggttttggt caaaatttcc 2820
   ggactgcgat tgtacatgct gttaacggct ccgcccacta ttaatgaaat taaaaattcc 2880
   aattttaaaa aacgcagcaa gagaaacatt tgtatgaaag aatgcgtaga aggaaagaaa 2940
   aatgtcgtcg acatgctgaa caacaagatt aatatgcctc cgtgtataaa aaaaatattg 3000
   aacgatttga aagaaaacaa tgtaccgcgc ggcggtatgt acaggaagag gtttatacta 3060
   aactgttaca ttgcaaacgt ggtttcgtgt gccaagtgtg aaaaccgatg tttaatcaag 3120
   gctctgacgc atttctacaa ccacgactcc aagtgtgtgg gtgaagtcat gcatctttta 3180
   atcaaatccc aagatgtgta taaaccacca aactgccaaa aaatgaaaac tgtcgacaag 3240
   ctctgtccgt ttgctggcaa ctgcaagggt ctcaatccta tttgtaatta ttgaataata 3300
   aaacaattat aaatgctaaa tttgtttttt attaacgata caaaccaaac gcaacaagaa 3360
   catttgtagt attatctata attgaaaacg cgtagttata atcgctgagg taatatttaa 3420
   aatcattttc aaatgattca cagttaattt gcgacaatat aattttattt tcacataaac 3480
   tagacgcctt gtcgtcttct tcttcgtatt ccttctcttt ttcatttttc tcctcataaa 3540
   aattaacata gttattatcg tatccatata tgtatctatc gtatagagta aattttttgt 3600
   tgtcataaat atatatgtct tttttaatgg ggtgtatagt accgctgcgc atagtttttc 3660
   tgtaatttac aacagtgcta ttttctggta gttcttcgga gtgtgttgct ttaattatta 3720
   aatttatata atcaatgaat ttgggatcgt cggttttgta caatatgttg ccggcatagt 3780
   acgcagcttc ttctagttca attacaccat tttttagcag caccggatta acataacttt 3840
   ccaaaatgtt gtacgaaccg ttaaacaaaa acagttcacc tcccttttct atactattgt 3900
   ctgcgagcag ttgtttgttg ttaaaaataa cagccattgt aatgagacgc acaaactaat 3960
   atcacaaact ggaaatgtct atcaatatat agttgctgat atcatggaga taattaaaat 4020
   gataaccatc tcgcaaataa ataagtattt tactgttttc gtaacagttt tgtaataaaa 4080
   aaacctataa atattccgga ttattcatac cgtcccacca tcgggcgcgg atccatggga 4140
   agtagccatc atcatcatca tcatctggaa gttctgttcc aggggcccgc agaattagaa 4200
   caccttgcac agaatatatc taaatcgcat ctggaaacct gccaatactt gagagaagag 4260
   ctccagcaga taacgtggca gaccttttta caggaagaaa ttgagaacta tcaaaacaag 4320
   cagcgggagg tgatgtggca attgtgtgcc atcaaaatta cagaagctat acagtatgtg 4380
   gtggagtttg ccaaacgcat tgatggattt atggaactgt gtcaaaatga tcaaattgtg 4440
   cttctaaaag caggttctct agaggtggtg tttatcagaa tgtgccgtgc ctttgactct 4500
   cagaacaaca ccgtgtactt tgatgggaag tatgccagcc ccgacgtctt caaatcctta 4560
   ggttgtgaag actttattag ctttgtgttt gaatttggaa agagtttatg ttctatgcac 4620
   ctgactgaag atgaaattgc attattttct gcatttgtac tgatgtcagc agatcgctca 4680
   tggctgcaag aaaaggtaaa aattgaaaaa ctgcaacaga aaattcagct agctcttcaa 4740
   cacgtcctac agaagaatca ccgagaagat ggaatactaa caaagttaat atgcaaggtg 4800
   tctacattaa gagccttatg tggacgacat acagaaaagc taatggcatt taaagcaata 4860
   tacccagaca ttgtgcgact tcattttcct ccattataca aggagttgtt cacttcagaa 4920
   tttgagccag caatgcaaat tgatgggtaa gaattccgga gcggccgctg cagatctgat 4980
   cctttcctgg gacccggcaa gaaccaaaaa ctcactctct tcaaggaaat ccgtaatgtt 5040
   aaacccgaca cgatgaagct tgtcgttgga tggaaaggaa aagagttcta cagggaaact 5100
   tggacccgct tcatggaaga cagcttcccc attgttaacg accaagaagt gatggatgtt 5160
   ttccttgttg tcaacatgcg tcccactaga cccaaccgtt gttacaaatt cctggcccaa 5220
   cacgctctgc gttgcgaccc cgactatgta cctcatgacg tgattaggat cgtcgagcct 5280
   tcatgggtgg gcagcaacaa cgagtaccgc atcagcctgg ctaagaaggg cggcggctgc 5340
   ccaataatga accttcactc tgagtacacc aactcgttcg aacagttcat cgatcgtgtc 5400
   atctgggaga acttctacaa gcccatcgtt tacatcggta ccgactctgc tgaagaggag 5460
   gaaattctcc ttgaagtttc cctggtgttc aaagtaaagg agtttgcacc agacgcacct 5520
   ctgttcactg gtccggcgta ttaaaacacg atacattgtt attagtacat ttattaagcg 5580
   ctagattctg tgcgttgttg atttacagac aattgttgta cgtattttaa taattcatta 5640
   aatttataat ctttagggtg gtatgttaga gcgaaaatca aatgattttc agcgtcttta 5700
   tatctgaatt taaatattaa atcctcaata gatttgtaaa ataggtttcg attagtttca 5760
   aacaagggtt gtttttccga accgatggct ggactatcta atggattttc gctcaacgcc 5820
   acaaaacttg ccaaatcttg tagcagcaat ctagctttgt cgatattcgt ttgtgttttg 5880
   ttttgtaata aaggttcgac gtcgttcaaa atattatgcg cttttgtatt tctttcatca 5940
   ctgtcgttag tgtacaattg actcgacgta aacacgttaa ataaagcttg gacatattta 6000
   acatcgggcg tgttagcttt attaggccga ttatcgtcgt cgtcccaacc ctcgtcgtta 6060
   gaagttgctt ccgaagacga ttttgccata gccacacgac gcctattaat tgtgtcggct 6120
   aacacgtccg cgatcaaatt tgtagttgag ctttttggaa ttatttctga ttgcgggcgt 6180
   ttttgggcgg gtttcaatct aactgtgccc gattttaatt cagacaacac gttagaaagc 6240
   gatggtgcag gcggtggtaa catttcagac ggcaaatcta ctaatggcgg cggtggtgga 6300
   gctgatgata aatctaccat cggtggaggc gcaggcgggg ctggcggcgg aggcggaggc 6360
   ggaggtggtg gcggtgatgc agacggcggt ttaggctcaa atgtctcttt aggcaacaca 6420
   gtcggcacct caactattgt actggtttcg ggcgccgttt ttggtttgac cggtctgaga 6480
   cgagtgcgat ttttttcgtt tctaatagct tccaacaatt gttgtctgtc gtctaaaggt 6540
   gcagcgggtt gaggttccgt cggcattggt ggagcgggcg gcaattcaga catcgatggt 6600
   ggtggtggtg gtggaggcgc tggaatgtta ggcacgggag aaggtggtgg cggcggtgcc 6660
   gccggtataa tttgttctgg tttagtttgt tcgcgcacga ttgtgggcac cggcgcaggc 6720
   gccgctggct gcacaacgga aggtcgtctg cttcgaggca gcgcttgggg tggtggcaat 6780
   tcaatattat aattggaata caaatcgtaa aaatctgcta taagcattgt aatttcgcta 6840
   tcgtttaccg tgccgatatt taacaaccgc tcaatgtaag caattgtatt gtaaagagat 6900
   tgtctcaagc tcgccgcacg ccgataacaa gccttttcat ttttactaca gcattgtagt 6960
   ggcgagacac ttcgctgtcg tcgacgtaca tgtatgcttt gttgtcaaaa acgtcgttgg 7020
   caagctttaa aatatttaaa agaacatctc tgttcagcac cactgtgttg tcgtaaatgt 7080
   tgtttttgat aatttgcgct tccgcagtat cgacacgttc aaaaaattga tgcgcatcaa 7140
   ttttgttgtt cctattattg aataaataag attgtacaga ttcatatcta cgattcgtca 7200
   tggccaccac aaatgctacg ctgcaaacgc tggtacaatt ttacgaaaac tgcaaaaacg 7260
   tcaaaactcg gtataaaata atcaacgggc gctttggcaa aatatctatt ttatcgcaca 7320
   agcccactag caaattgtat ttgcagaaaa caatttcggc gcacaatttt aacgctgacg 7380
   aaataaaagt tcaccagtta atgagcgacc acccaaattt tataaaaatc tattttaatc 7440
   acggttccat caacaaccaa gtgatcgtga tggactacat tgactgtccc gatttatttg 7500
   aaacactaca aattaaaggc gagctttcgt accaacttgt tagcaatatt attagacagc 7560
   tgtgtgaagc gctcaacgat ttgcacaagc acaatttcat acacaacgac ataaaactcg 7620
   aaaatgtctt atatttcgaa gcacttgatc gcgtgtatgt ttgcgattac ggattgtgca 7680
   aacacgaaaa ctcacttagc gtgcacgacg gcacgttgga gtattttagt ccggaaaaaa 7740
   ttcgacacac aactatgcac gtttcgtttg actggtacgc ggcgtgttaa catacaagtt 7800
   gctaacgtaa tcatggtcat agctgtttcc tgtgtgaaat tgttatccgc tcacaattcc 7860
   acacaacata cgagccggaa gcataaagtg taaagcctgg ggtgcctaat gagtgagcta 7920
   actcacatta attgcgttgc gctcactgcc cgctttccag tcgggaaacc tgtcgtgcca 7980
   gctgcattaa tgaatcggcc aacgcgcggg gagaggcggt ttgcgtattg ggcgctcttc 8040
   cgcttcctcg ctcactgact cgctgcgctc ggtcgttcgg ctgcggcgag cggtatcagc 8100
   tcactcaaag gcggtaatac ggttatccac agaatcaggg gataacgcag gaaagaacat 8160
   gtgagcaaaa ggccagcaaa aggccaggaa ccgtaaaaag gccgcgttgc tggcgttttt 8220
   ccataggctc cgcccccctg acgagcatca caaaaatcga cgctcaagtc agaggtggcg 8280
   aaacccgaca ggactataaa gataccaggc gtttccccct ggaagctccc tcgtgcgctc 8340
   tcctgttccg accctgccgc ttaccggata cctgtccgcc tttctccctt cgggaagcgt 8400
   ggcgctttct catagctcac gctgtaggta tctcagttcg gtgtaggtcg ttcgctccaa 8460
   gctgggctgt gtgcacgaac cccccgttca gcccgaccgc tgcgccttat ccggtaacta 8520
   tcgtcttgag tccaacccgg taagacacga cttatcgcca ctggcagcag ccactggtaa 8580
   caggattagc agagcgaggt atgtaggcgg tgctacagag ttcttgaagt ggtggcctaa 8640
   ctacggctac actagaagga cagtatttgg tatctgcgct ctgctgaagc cagttacctt 8700
   cggaaaaaga gttggtagct cttgatccgg caaacaaacc accgctggta gcggtggttt 8760
   ttttgtttgc aagcagcaga ttacgcgcag aaaaaaagga tctcaagaag atcctttgat 8820
   cttttctacg gggtctgacg ctcagtggaa cgaaaactca cgttaaggga ttttggtcat 8880
   gagattatca aaaaggatct tcacctagat ccttttaaat taaaaatgaa gttttaaatc 8940
   aatctaaagt atatatgagt aaacttggtc tgacagttac caatgcttaa tcagtgaggc 9000
   acctatctca gcgatctgtc tatttcgttc atccatagtt gcctgactcc ccgtcgtgta   9060
   gataactacg atacgggagg gcttaccatc tggccccagt gctgcaatga taccgcgaga   9120
   cccacgctca ccggctccag atttatcagc aataaaccag ccagccggaa gggccgagcg   9180
   cagaagtggt cctgcaactt tatccgcctc catccagtct attaattgtt gccgggaagc   9240
   tagagtaagt agttcgccag ttaatagttt gcgcaacgtt gttgccattg ctacaggcat   9300
   cgtggtgtca cgctcgtcgt ttggtatggc ttcattcagc tccggttccc aacgatcaag   9360
   gcgagttaca tgatccccca tgttgtgcaa aaaagcggtt agctccttcg gtcctccgat   9420
   cgttgtcaga agtaagttgg ccgcagtgtt atcactcatg gttatggcag cactgcataa   9480
   ttctcttact gtcatgccat ccgtaagatg cttttctgtg actggtgagt actcaaccaa   9540
   gtcattctga gaatagtgta tgcggcgacc gagttgctct tgcccggcgt caatacggga   9600
   taataccgcg ccacatagca gaactttaaa agtgctcatc attggaaaac gttcttcggg   9660
   gcgaaaactc tcaaggatct taccgctgtt gagatccagt tcgatgtaac ccactcgtgc   9720
   acccaactga tcttcagcat cttttacttt caccagcgtt tctgggtgag caaaaacagg   9780
   aaggcaaaat gccgcaaaaa agggaataag ggcgacacgg aaatgttgaa tactcatact   9840
   cttccttttt caatattatt gaagcattta tcagggttat tgtctcatga gcggatacat   9900
   atttgaatgt atttagaaaa ataaacaaat aggggttccg cgcacatttc cccgaaaagt   9960
   gccacctgac gtctaagaaa ccattattat catgacatta acctataaaa ataggcgtat  10020
   cacgaggccc tttcgtctcg cgcgtttcgg tgatgacggt gaaaacctct gacacatgca  10080
   gctcccggag acggtcacag cttgtctgta agcggatgcc gggagcagac aagcccgtca  10140
   gggcgcgtca gcgggtgttg gcgggtgtcg gggctggctt aactatgcgg catcagagca  10200
   gattgtactg agagtgcacc atatgcggtg tgaaataccg cacagatgcg taaggagaaa  10260
   ataccgcatc aggcgccatt cgccattcag gctgcgcaac tgttgggaag ggcgatcggt  10320
   gcgggcctct tcgctattac gccagctggc gaaaggggga tgtgctgcaa ggcgattaag  10380
   ttgggtaacg ccagggtttt cccagtcacg acgttgtaaa acgacggcca gtgcc        10435

## Description

The present invention relates to an isolated protein comprising a method for screening for a ligand for a ligand binding domain of a nuclear receptor using a test system comprising an isolated protein comprising a ligand binding domain of a nuclear receptor.

Nuclear receptors represent a superfamily of proteins which are found within cells and which induce signals of ligands such as hormones and vitamins. In response, agonist-activated nuclear receptors usually increase expression of specific genes upon activation in general together with other proteins.

Thus, nuclear receptors act as agonist-induced transcription factors which directly interact as monomers, homodimers or heterodimers with DNA response element of target genes as well as through signaling pathways. In contrast to membrane receptors and membrane-associated receptors, nuclear receptors reside within cells, either in cytoplasm or in the nucleus. Thus, nuclear receptors comprise a class of intercellular, soluble, ligand-regulated factors which are found in eukaryotic cells. Nuclear receptors have the ability to directly bind to DNA and regulate the expression of adjacent genes; hence these receptors are classified as transcription factors. As detailed above, the regulation of gene expression by nuclear receptor is ligand-dependent, wherein nuclear receptors are normally only active in the presence of an agonist. Ligand binding to a nuclear receptor results in a conformational change in the receptor, which in turn activates the receptor resulting in general in up-regulation of gene expression.

Due to their unique ability to directly interact with and control the expression of genomic DNA, nuclear receptors play a key role in development and homeostasis of organisms.

The members of the superfamily of nuclear receptors display an overall structural motif of four modular domains:
- A variable amino-terminal domain (also referred to as N-terminal regulatory domain), which contains activation function 1 (AF-1), whose action is independent of the presence of a ligand. The transcriptional activation of AF-1 is normally weak, but synergizes with AF-2 to up- regulate gene expression. This domain is highly variable in sequence between various nuclear receptors.
- A highly conserved DNA-binding domain (DBD) contains two zinc fingers and binds to hormone response elements (HREs).
- A less conserved ligand binding domain (LBD), though only moderately conserved in sequence, is highly conserved in structure among the various nuclear receptors. The structure of the LBD is referred to as an alpha-helical sandwich fold. The ligand binding cavity is within the interior of the LBD just below three anti-parallel alpha helices forming the "sandwich filling". Along with the DBD, the LBD contributes to the dimerization interface of the receptor and, in addition, binds co-activator and co-repressor proteins. Additionally, it contains the activation function 2 (AF-2), whose activation is dependent on the presence of bound ligand and which synergizes with AF-1 (see above).
- A variable carboxy-terminal domain which is variable in sequence between various nuclear receptors.

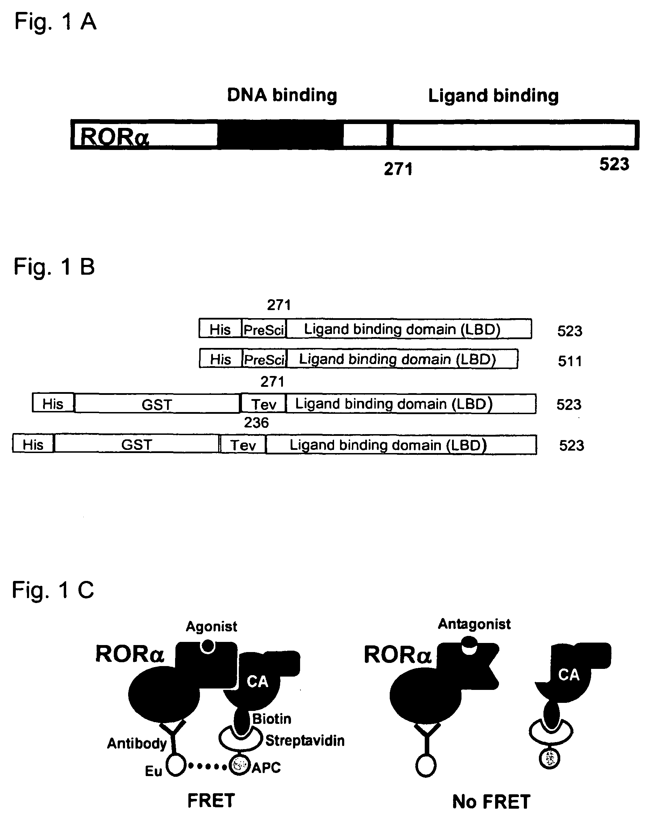

As an example, the structure of RORα1 is shown in Figure 1A.

Depending on their mechanism of action and subcellular distribution in the absence of ligand, nuclear receptors (NRs) are classified into four classes.

Type I NRs are nuclear receptors located in the cytosol. Binding of a ligand to type I NRs results in dissociation of heat shock proteins, homo-dimerization, translocation to the nucleus and binding to HREs consisting of two half sites separated by variable length of DNA and the second half site having a sequence inverted from the first (inverted repeat). After formation of a nuclear receptor/DNA complex, other proteins are recruited which transcribe DNA downstream from the HRE into mRNA and, eventually, a protein which causes a change in cell function.

Type II NRs remain in the nucleus in the presence and absence of a ligand. They bind as heterodimers (usually with RXR) to DNA. In the absence of a ligand, type II NRs are often complexed with co-repressor proteins. Ligand binding to the nuclear receptor causes dissociation of co-repressors and recruitment of co-activator proteins and further proteins including RNA polymerase, which effects translation of DNA into mRNA.

Type III nuclear receptors are similar to type I NRs, but bind to direct repeat instead of inverted repeat HREs.

Type IV NRs bind either as monomers or dimers, but only a single DNA binding domain of the receptor binds to a half site HRE.

As detailed above, nuclear receptors activated upon ligand binding and bound to HREs recruit a significant number of other proteins which modify transcription of the associated target gene into mRNA. The function of these transcription co-regulators are varied and include chromatin remodeling in order to render the target gene more or less accessible to transcription, or a bridging function to stabilized the binding of other co-regulatory proteins. The co-regulatory protein (also referred to as co-factor) may be a co-activator, which often has an intrinsic histone acetyltransferase (HAT) activity which weakens the association of histones to DNA and, therefore, promotes transcription. In contrast thereto, co-repressors, which are preferably bound upon the binding of an agonist to NR, recruit histone deacetylases (HDACs), which promotes the association of histones to DNA and, therefore, represses transcription.

Members of the nuclear receptor superfamily include receptors such as those for glucocorticoids (GRs), androgens (ARs), mineralocorticoids (MRs), progestins (PRs), estrogens (ERs), thyroid hormones (TRs), vitamin D (VDRs), retinoids (RARs and RXRs), peroxisomes (XPARs and PPARs) and icosanoids (IRs).

Due to their role in development and homeostasis, nuclear receptors are an interesting target for studying their involvement in particular functions. Additionally, some of the nuclear receptors are so-called "orphan receptors", whose natural ligand is still unknown. Accordingly, it is of particular interest to identify these yet unknown natural ligands. Additionally, due to their involvement in physiological and pathophysiological functions of the body, nuclear receptors are an interesting target in pharmacological sciences. Data on functional interactions between nuclear receptors and co-regulators offer new chances in the development of novel pharmaceutical therapies for a wide range of diseases. Clinical strategies addressing the role of co-activators and co-repressors involved in cell proliferation with steroid receptors, may offer new treatments for, e.g. cancer. Furthermore, the functional importance of co-regulators and signaling receptors involved in energy metabolism may offer new opportunities for diseases with impaired energy metabolism.

WO 03/093312 A1 discloses the crystal structure of the ligand binding domain of the retinoic acid-related orphan alpha. The document provides a three-dimensional model of the retinoic acid-related orphan alpha and means for design of modulators of the retinoic acid-related orphan alpha. A sequence is disclosed which is identical to SEQ ID NO: 2 of the present invention over a large region. The peptide is used for screening for ligands and medicaments. However, no test system is disclosed where a protein, a ligand or a candidate ligand and a co-factor are combined.

WO 03/000732 A2 refers to polypeptides derived from retinoic acid-related orphan receptor and their applications. A peptide is disclosed with identity over a large region to SEQ ID NO: 2 of the present invention. The peptide is used for screening for a ligand which is an agonist, antagonist or co-factor of said peptide. There is no disclosure to render the binding of a co-factor dependent on the binding of a ligand and to measure ligand binding via the binding of the co-activator.

However, it was not possible to isolate proteins comprising a ligand binding domain of a nuclear receptor in a controllable form, particularly not for RORalpha.

Surprisingly, the inventor succeeded in providing an isolated protein comprising a ligand binding domain of a nuclear receptor in a controllable form. The protein could be prepared by culturing a cell comprising a nucleic acid coding for the protein under suitable conditions and isolating the protein from the cell culture. Thereafter, the isolated protein was contacted with a detergent, particularly lithium dodecyl sulphate (LDS), which restored controllability of the isolated protein. "Controllable form" means that the ligand binding domain is activatable upon binding of an agonist to the ligand binding domain.

The present invention refers to a method for screening for a ligand for a ligand binding domain of a nuclear receptor comprising the steps of:
a) contacting a test system comprising
   - an isolated protein comprising a ligand binding domain of a nuclear receptor wherein the ligand binding domain is activatable upon binding of an agonist to the ligand binding domain,
   - a co-factor werein the co-factor is bound by the nuclear receptor upon binding of a ligand, and
   - means for detecting the interaction between the protein and the co-factor upon binding of a ligand, especially an agonist, to the ligand binding domain of the nuclear receptor with a substance and
b) detecting a measurable signal upon binding of the substance to the ligand binding domain, thereby identifying the substance as a ligand for the ligand binding domain,
wherein the binding of the substance to the ligand binding domain induces the binding of the co-factor to the protein and the binding of the co-factor to the protein induces the measurable signal.

The present invention discloses and uses an isolated protein comprising a ligand binding domain of a nuclear receptor wherein the ligands binding domain is activatable upon binding of an agonist to the ligand binding domain.

The ligand binding domain (see also above) of a nuclear receptor is that domain of the nuclear receptor which acts in response to ligand binding, which causes a conformational change in the nuclear receptor to induce a response, thereby acting as a molecular switch to turn on transcriptional activity. The ligand binding domain is a flexible unit, wherein the binding of a ligand stabilizes its conformation which in turn favors co-factor binding to modify receptor activity. The co-activator may bind to the activator function 2 (AF-2) at the same terminal end of the ligand binding domain. The binding of different ligands may alter the conformation of the ligand binding domain, which ultimately affects the DNA-binding specificity of the DNA binding domain of the nuclear receptor. The ligand binding domains of various nuclear receptors are well known in the art and are summarized, for example, at EMBL-EBI (www.ebi.ac.uk) or InterPro: IPR000536 (see http://srs.ebi.ac.uk/srsbin/cgi. bin/wgetz?[interpro-AccNumber: IPR000536]+-e).
- amino acids 325 to 318 of retinoic acid receptor-related orphan receptor gamma (ROR gamma)
- amino acids 192 to 464 of hepatocyte nuclear factor alpha 1 (HNF4 alpha 1)
- amino acids 192 to 474 of hepatocyte nuclear factor alpha 2 (HNF4 alpha 2)
- amino acids 233 to 423 of estrogen-related receptor alpha (ERR alpha)
- amino acids 248 to 500 of estrogen-related receptor beta (ERR beta)
- amino acids 250 to 435 of estrogen-related receptor gamma (ERR gamma)

The nuclear receptor may be any known nuclear receptor. Depending on their sequence homologies nuclear receptors are divided into seven subfamilies.

Subfamily 1 includes thyroid hormone receptor-like, including thyroid hormone receptor-α and -β, retinoic acid receptor-α, -β and -γ, peroxisome proliferators-activated receptor-α, -β/δ, γ, Rev-ErbA-α and -β, RAR-related orphan receptors α, β and γ, liver X receptor-like α and β, famesoid X receptor, vitamin D receptor, pregnane X receptor and constitutive androstane receptor.

Subfamily 2 relates to retinoic X receptor-like including, for example, hepatocyte nuclear receptor-4 (α and γ), retinoic X receptor (α, β and γ), testicular receptor (2 and 4), human homologue of the Drosophila tailless gene, photoreceptor cell-specific nuclear receptor, chicken ovalbumin upstream promoter-transcription factor (I and II) and V-erbA-related.

Subfamily 3 relates to estrogen receptor-like including, amongst others, estrogen receptor (α and β), estrogen related receptor (α, β and γ), corticoid receptor, mineralocorticoid receptor, progesterone receptor and androgen receptor.

Subfamily 4 relates to nerve growth factor IB-like including receptors such as nerve growth factor IB, nuclear receptor related 1 and neuron-derived orphan receptor 1.

Subfamily 5 relates to steroidogenic factor-like including, for example, steroidogenic factor 1 and liver receptor homolog-1.

Subfamily 6 relates to germ cell nuclear factor- like including germ cell nuclear factor.

A further subfamily, referred to as subfamily 0, includes miscellaneous receptors such as dosage-sensitive sex reversal, adrenal hypoplasia critical region, on chromosome X, gene 1 (DAXI), small heterodimer partner and nuclear receptors with two DNA binding domains (2DBD-NR).

According to the present invention, the ligand binding domain of the nuclear receptor is comprised in an isolated protein. An isolated protein in the context of the present invention relates to a protein which is not in its natural environment. Accordingly, the "isolated protein" is not associated with proteins, it is normally found within nature or is isolated from a cell in which it normally occurs or is isolated from a cell in which the nucleic acid coding for the same has been expressed or is essentially free from other proteins from the same cellular source. The protein may be a naturally occurring protein, preferably a naturally occurring nuclear receptor or part thereof, wherein the part encompasses the ligand binding domain. However, the protein may also be artificial in that it does not naturally occur or in that it may encompass one or more sections which are naturally not connected to the ligand binding domain, for example, a fusion protein comprising or consisting of a ligand binding domain of a nuclear receptor and a further protein such as a second domain used for, e.g., purification or detection purposes.

Preferably, the term "isolated protein" means a protein molecule which is essentially separated from other cellular components of its natural environment. However, after isolation of the protein, cellular components may be added again, e.g., for measuring signal transduction pathways. Additionally, the skilled person will understand that the isolated protein is to be kept under suitable conditions allowing activity of the isolated protein, e.g., suitable buffers, pH values, ions, etc.

"Controllable form" in the context of the isolated protein of the invention comprising a ligand binding domain of a nuclear receptor relates to a protein, which is still amendable to activation upon agonist binding to the ligand binding domain. As detailed above, the LBD is activated upon binding of an agonistic ligand to the same, which alters gene expression of a target gene. However, up to now it was not possible to produce RORalpha protein or many other isolated proteins comprising an LBD of a nuclear receptor which could be controlled or regulated, i.e. there was no significant or only little difference of activity in the presence or absence of an agonistic ligand for the respective LBD.

Accordingly, an isolated protein in controllable form, as defined above can be detected by comparing activity in the presence or absence of an agonistic ligand for the respective LBD. Activity of the LBD may be determined in any suitable matter, e.g., by determining influence on the downstream elements of the respective signal transduction pathway, such as binding to any of the downstream components of the respective signal transduction pathway, such as co-regulator and/or target DNA. An example of such a task is described in the Example 2 and illustrated in Figure 1C.

Preferably, the activity of the isolated protein comprising an LBD of an NR in controllable form amounts to at least 1.2, more preferably at least 1.5, still more preferably at least 2, 3, 4 or 5, and most preferably at least 10, if the activity in the presence of an agonistic ligand is compared to that in the absence of an agonistic ligand for the respective LBD,
An isolated protein particularly relates to an isolated protein comprising a ligands binding domain of an NR in controllable form, wherein the protein is not constitutively active, which means that the protein is not active in the absence of an agonistic ligand for the respective LBD.

In one embodiment of the invention the isolated protein may comprise or consist of the full amino acid sequence of a naturally occurring nuclear receptor. Alternatively, the isolated protein may comprise or consist of a part of a naturally occurring nuclear receptor, provided that the LBD is still present in the part of the nuclear receptor.

The isolated protein may comprise or consist of any of the nuclear receptors as defined above. The nuclear receptor may be the isolated protein of it may be fused to a further domain, e.g., in order to ease purification of the protein or to detect the protein or to measure activity of the protein.

As detailed above, the isolated protein may also comprise or consist of a part of the nuclear receptor as long as the LBD of the nuclear receptor is part of the protein. Accordingly, the isolated protein may also comprise the amino terminal regulatory domain, the DNA binding domain, a hinge region connecting the DNA binding domain and the ligand binding domain, and/or a carboxy-terminal domain of a nuclear receptor. The additional domains and regions may independent from each other, be derived from the same nuclear receptor as the LBD or from one or more other nuclear receptors.

In a preferred embodiment of the invention, the nuclear receptor is a retinoic acid receptor-related orphan receptor (ROR), particularly RORα, RORβ or RORγ, especially RORα.

The orphan receptors ROR, also referred to as RZR, constitute a subfamily of nuclear receptors for which initially no ligand had been identified. Presently, three subtypes of ROR receptors have been identified - RORα, RORβ and RORγ. ROR receptors bind in monomeric or dimeric form, each to a specific response element consisting of a sequence rich in A/T preceding a sequence of the PuGGTCA type and modulate transcription of the target genes.

Following alternative splicing, the RORα gene leads to four isoforms α1, α2, α3 and α4 RZRA, which differ in their N-terminal domain and show DNA recognition and distinct transactivation properties.

As for nuclear receptors, any mammalian ROR receptor is preferred, and human ROR receptors are even more preferred.

RORα (also referred to as RAR-related orphan receptor A, RZRA, ROR1, ROR2, ROR3, NR1F1) has been sequenced, and its sequence is available from the NCBI (National Center for Biotechnology Information) data bank under accession no. U04897, which provides the human mRNA and protein sequence. Known agonistic ligands for RORα include cholesterol, derivatives thereof and possibly melatonin.

RORβ (also referred to as RAR-related orphan receptor B, RZRB, NR1F2) has been sequenced and its sequence is available from the NCBI (National Center for Biotechnology Information) data bank under accession no. Y08639, which provides the human mRNA and protein sequence. A known agonistic ligand for RORβ is retinoic acid.

RORγ (also referred to as RAR-related orphan receptor C, RZRG, RORG, NR1F3, TOR) has been sequenced and its sequence is available from the NCBI (National Center for Biotechnology Information) data bank under accession no. U16997, which provides the human mRNA and protein sequence.

The three forms of ROR fulfill a number of critical roles including:
- RORα: development of the cerebellum, maintenance of bone, lymph node development, immune response, development of skeletal muscle, differentiation of smooth muscle cells, lipid metabolism (diseases: e.g. cerebellar degeneration, osteoporosis, ischemia-induced angiogenesis, artherosclerosis, inflammatory diseases)
- RORβ: central nervous system
- RORγ: immune response, skeletal muscle, adipocyte differentiation
Particularly preferred is an isolated protein comprising a ligand binding domain of RORα in a controllable form. The full length protein of RORα consists of 523 amino acids, wherein amino acids 271 - 523 code for the ligand binding domain. A particularly preferred protein is shown in SEQ ID NO. 1:

An exemplary sequence comprising the above domain as well as a tag and a cleavage site is shown in the following: reads as follows:

The isolated protein encompasses the domain of SEQ ID NO: 1 with a His-tag (HHHHHH; SEQ ID NO: 3) and PreScission cleavage site (LEVLFQGP; SEQ ID NO: 4) inserted at amino acid 270 of RORα1. However, the His-tag may be substituted with another suitable tag e.g. as described herein as well as with another suitable cleavage site e.g. as described below. Examples of those are shown in Fig. 1B.

In one embodiment of the present invention, the isolated protein comprises a marker, particularly a tag.

A marker in the context of the present invention may be any kind of molecule which can be easily detected. In the present invention, the molecule is bound to the isolated protein, therefore, the presence of the marker is indicative for the presence of the isolated protein. Markers (also referred to as labels) are known to a skilled person and include, for example, radiolabels (such as ³H, ³²P, ³⁵S or ¹⁴C), fluorescence markers (such as fluorescein, green fluorescence protein, or DyLight 488), enzymes (such as horse radish oxidase, β-lactamase, alkaline phosphatase or β-glucosidase) or an antigene detectable by a suitable antibody or antibody fragment.

Preferably, the marker is a tag. Tags are usually proteins which are used as biochemical indicators. They may be included into a protein, such as a recombinant, expressed protein and can serve several purposes. Preferably, they are used for purifying the proteins to which they are attached using standard conditions suitable for the particular tag, However, the tags may be also used as indicators in order to detect the presence of a particular protein.

A number of (affinity) tags are known at present. These are usually divided into 3 classes according to their size: small tags have a maximum of 12 amino acids, medium-sized ones have a maximum of 60 and large ones have more than 60. The small tags include the Arg-tag, the His-tag, the Strep-tag, the Flag-tag, the T7-tag, the V5-peptide-tag and the c-Myc-tag, the medium-sized ones include the S-tag, the HAT-tag, the calmodulin-binding peptide, the chitin-binding peptide and some cellulose-binding domains. The latter can contain up to 189 amino acids and are then regarded, like the GST- (glutathione-S-transferase-) and MBP-tag (maltose binding protein-tag), as large affinity tags.

In order to produce especially pure proteins, so-called double tags or tandem tags were developed. In this case the proteins are purified in two separate chromatography steps, in each case utilizing the affinity of a first and then of a second tag. Examples of such double or tandem tags are the GST-His-tag (glutathione-S- transferase fused to a polyhistidine-tag), the 6xHis-Strep-tag (6 histidine residues fused to a Strep-tag), the 6xHis-tag100-tag (6 histidine residues fused to a 12-amino-acid protein of mammalian MAP-kinase 2), 8xHis-HA-tag (8 histidine residues fused to a haemagglutinin-epitope-tag), His-MBP (His-tag fused to a maltose-binding protein, FLAG-HA-tag (FLAG-tag fused to a hemagglutinin-epitope-tag), and the FLAG-Strep-tag.

Preferably, the isolated protein of the present invention comprises a tag selected from the group consisting of His-tag, Arg-tag, Strep-tag, Flag-tag, T7-tag, V5-peptide-tag, c-Myc-tag, S-tag, HAT-tag, calmodulin-binding peptide-tag, chitin-binding peptide-tag, GST-tag and MBP-tag. However, any other tag may be also used, but some tags such as His-tag, Arg-tag, Strep-tag, Flag-tag or GST-tag are preferred.

In an embodiment of the invention the isolated protein comprises a marker or tag, wherein the marker or tag is removable from the protein by proteolytic cleavage at a specific cleavage site, for example a cleavage site for an enzyme. This may be located between the LBD and the marker or tag. The cleavage site could for example be a protease cleavage site. Examples of proteases are chymotrypsin, trypsin, elastase, and plasmin; the corresponding cleavage sites are known to a person skilled in the art. Since the molecule to be purified is a protein, specific proteases, especially proteases from viruses that normally attack plants, are preferred. Examples of suitable specific proteases are thrombin, factor Xa, Igase, TEV-protease from the "Tobacco Etch Virus", the protease PreScission (Human Rhinovirus 3C Protease), enterokinase or Kex2. TEV-protease and PreScission are especially preferred.

An example of a protein comprising an LBD, a His-tag and a precision cleaving site is disclosed in SEQ ID NO. 2. A suitable nucleic acid and a vector encoding that protein are given in SEQ ID NO: 5 and SEQ ID NO: 6, respectively. Additionally, exemplary isolated proteins of the invention are illustrated in Figure 1B.

### Nucleotides 4021 to 5040 of the vector of SEQ ID NO: 6:

- Upper nucleic acid sequence: coding strand (SEQ ID NO: 5)
- Lower nucleic acid sequence: template strand
- Amino acid sequence: LBD (as defined in SEQ ID NO: 1) with His-tag and PreScission cleavage site (SEQ ID NO: 2)
- □: cloning sites (as specified)
- *ATG* and *TAA:* start/stop (each adjacent to cloning site)
- CATCATCATCATCATCATCTGGAAGTTCTGTTCCAGGGGCCC: His-tag and PreScission cleavage site

### Vector (for details see above) (SEQ ID NO: 6):

------ : vector insert

The present invention discloses a method of producing the isolated protein comprising the steps of:
a) cultuuing a cell comprising a nucleic acid coding for the isolated protein under conditions conducive to the production of the protein,
b) isolating the protein from the cell culture, and
c) contacting the isolated protein of step b) with a detergent, particularly with an alkaline salt of a saturated unbranched C6-C20 alkyl sulphate or carbonate or with an isoprenyl salt.
In general, steps a) and b) of the above method method are well known to the skilled person. However, in the following, they will be briefly summarized and illustrated by examples. The skilled person will understand that the described method may be modified depending on the respective protein of the invention to be isolated, the cell used for cell culture, etc.

For the production of recombinant protein, i.e. for synthesis of an exogene gene product in a living cell, a multiplicity of expression systems is available. These include series of well-known organisms and cell lines (bacteria, insect cells, yeasts, mammalian cells, etc.) as well as various expression vectors with different promoters, selection markers and optionally fusion partners. The production of a recombinant protein usually includes the introduction of the coding gene into a plasmid or any other suitable vector. In general, the selection of a suitable vector or plasmid depends on the intended host cell. This vector is then introduced into the chosen target cell (transformation or transfection) and the target cell is cultivated. Depending on the promoter, the expression of gene may occur throughout a period of cultivation or may be induced by an external signal.

As detailed above, it may be necessary to produce a suitable nucleic acid coding for the isoled protein or a vector containing the same.

The nucleic acid coding for the isolated protein may be a naturally occurring gene coding for a nuclear receptor or it may be a part thereof coding for a part of the nuclear receptor as defined above. The isolation of nucleic acids coding for naturally occurring proteins or a part thereof is well known to the skilled person and may include the isolation using suitable probes and separation methods, or the nucleic acid may be derived from a commercial supplier. If a nucleic acid is used which is not comprised by a naturally occurring gene (e.g., nucleic acid coding for a fusion protein comprising a full or partial NR and a tag) the respective nucleic acid coding for the fusion protein may be produced in accordance with standard procedures including well-known methods of genetic engineering. Usually, suitable restriction endonucleases are used to cut DNA at specific sites. The fragments formed by restriction enzymes may be joined together with ligase. Thereafter, the DNA may be introduced into a vector suitable for transferring genetic material into a cell. The vector may be a viral vector or plasmid vector. Suitable vectors include adenovirus, adeno-associated virus, cytomegalovirus, etc. Examples of comerially available vectors are pBR322, the pUC series, pBluescript, pTZ, pSP and pGEM. Alternatively, also naked DNA may be introduced into a cell. If the nucleic acid, i.e. DNA or RNA, is used without a vector, transfection may be carried out by mixing it with a cationic lipid to produce liposomes, which fuse with the cell membrane and deposit the nucleic acid inside the cell. The transfection may be also carried out by calcium phosphate, wherein, e.g., HEPES-buffered saline solution containing phosphate ions is combined with calcium chloride solution containing the DNA to be transfected. When the two solutions are combined, a fine precipitate of calcium phosphate is formed, binding the DNA to be transfected on its surface. The suspension of the precipitate is then added to the cell to be transfected (usually a cell culture grown in a monolayer). Other methods for transfection include electroporation, heat shock, magnetofection, nucleofection and the use of transfection agents such as Lipofectamine, Fugene, etc. A further approach is the "gene gun", where the DNA is coupled to a nanoparticle of an inert solid (usually gold) which is then shot directly into the target cells.

The transfection of a cell may be transient or stable. In case the nucleic acid introduced into a cell during a transfection process, is not inserted into the nuclear genome, the foreign nucleic acid may be lost at a later stage, when the cell undergoes mitosis. This is called transient transfection. More preferably, the transfection is a stable transfection, wherein the nucleic acid remains in the genome of the cell. In order to accomplish stable transfection, usually selection techniques are used, wherein the nucleic acid is co-transfected with another gene, providing for selective selection. The additional gene may confer resistance towards a certain condition or substance, e.g. an antibiotic or metabolic deficiency. Examples of suitable genes include neomycin resistant gene, hygromycin phosphotransferase gene, etc.

After introduction of the nucleic acid coding the protein of the invention into the host, the cell is grown under suitable conditions. A series of different host cells for the production of proteins is known to the skilled person including bacteria, insect cells, yeasts and mammalian cells. Examples of such cells are Sf9, Sf21, HEK 293 cells, CHO cells, HeLa cells, CaCo cells or NIH 3T3 cells.

The host cell may be either a primary cell or it may be a cell line, wherein cell lines are preferred.

The cell comprising the nucleic acid of the invention is grown and maintained under conditions conducive to the production of the cell. This includes an appropriate temperature and gas mixture (typically 37 °C, 5 % CO₂), optionally in a cell incubator. Culture conditions may vary widely for each cell type and are known to the skilled practitioner. The expression may take place for example in insect cells after transformation with suitable baculovirus vector systems. In such a case the temperature is kept at 26 °C wheras control of CO₂ is not required.

Aside from temperature and gas mixture, the most commonly varied factor in cell culture systems is the growth medium. Recipes for growth media can vary in pH, glucose concentration, growth factor and the presence of other nutrient components among others. Growth factors used for supplement media are often derived from animal blood such as calf serum.

A skilled person in the art knows how to derive a nucleic acid sequence, which may be DNA or RNA, from a protein sequence, taking into account the genetic code. He also knows how to produce such a nucleic acid sequence using standard techniques of molecular biology. This can be accomplished, for example, by the use and combination of existing sequences using restriction enzymes. The nucleic acid suitably also contains further elements, e.g., a promoter and a transcription start and stop signal and a translation start and stop signal.

After step a), the protein is isolated from the cell culture by any suitable separation or purification method known to the skilled person. If a sufficient amount of the target protein has been secreted into the medium, the isolation can continue with the same. Otherwise, it may be necessary to disrupt the cells. This can be effected, for example, by lysis of the cell, e.g., by means of ultrasound or hypertonic medium or by shearing. To remove insoluble components, the sample can, for example, be centrifuged, especially at 10,000 x g to 15,000 x g and the supernatant obtained can be used for step c) or may be further purified or concentrated.

The isolation of step b) may alternatively or additionally include well-known purification concentration steps such as extraction, precipitation, electrophoretic methods, chromatographic methods, etc. Examples of those include cell electrophoresis, ion exchange chromatography, size exclusion chromatography, SDS-PAGE chromatography, or affinity chromatography particularly immobilized metal ion affinity chromatography (IMAC).

Affinity purification is particularly envisioned, if the isolated protein of the invention comprises a suitable marker or tag, as defined above. Affinity purification is a special form of adsorption purification, in which there are, on a carrier, groups (binding partners) with high affinity and therefore high binding strength to one of the two domains, so that these can be adsorbed preferentially and thus separated from other substances. Purification can be carried out using a first and a second tag (e.g His-tag and GST-Tag). Purification takes place by specific binding to a suitable binding partner. The binding partner is preferably bound to a solid phase. The solid phase can be usual carrier materials, for example Sepharose, Superflow, Macroprep, POROS 20 or POROS 50. Separation is then carried out for example chromatographically, e.g. by gravity, HPLC or FPLC. The protein of interest may be eluted from the solid phase by altering the conditions, so that the changed conditions no longer permit binding between affinity marker or tag and binding partner (e.g. alteration of the pH value or the ionic strength), or by separating the molecule from the domain bound to the binding partner. Separation can be effected by cleavage of the bond between molecule and binding partner, e.g. by chemical means or using specific enzymes, as was described in detail above. Alternatively, it is also possible to use specific competitors, which are added in excess. Alternatively, the binding partner can also be bound to beads, especially magnetic beads. After adding the beads to the sample, binding takes place between the particular domain and the corresponding binding partner. The suspension can then be centrifuged for example, so that the labeled molecule sediments with the bead, and other components remain in the supernatant, from where they can be removed. Alternatively, the suspension is separated utilizing the magnetic properties of the beads. In one embodiment, the suspension is applied to a column, which is located in a magnetic field. As the magnetic beads and the molecule bound to them are retained in the magnetic field, other constituents of the sample can be washed out in several washing operations. The molecule of interest can then for example be washed from the beads using a suitable elution buffer, or can be separated from the beads by enzymatic cleavage e.g. at the cleavage site between the LBD and the tag or marker.

After step b), the isolated protein is contacted with a detergent, in order to provide an LBD in a controllable form. Detergents in the biological sense are membrane-active substances commonly used to disrupt the bipolar lipid membrane of cells in order to free and solubilize membrane-bound proteins. The value of the detergents is derived from their amphophilic nature. Each detergent molecule is characterized by a hydrophilic "head" region and a hydrophobic "tail" region. The result of this characteristic is the formation of a thermodynamically stable micelle with hydrophobic course in aqueous media. The hydrophobic core provides an environment that allows for the dissolution of hydrophobic molecules or domains of proteins. The detergent can be anionic, cationic, zwitterionic or non-ionic. Anionic and cationic detergents typically modify protein structure to a greater extent than the other two classes. The degree of modification varies with the individual protein and the particular detergent. Ionic detergents are also more sensitive to pH, ionic strength and the nature of the counter ion and can interfere with charge based analytical methods. Alternatively, most non-ionic detergents are non-denaturating, but are less effective at disrupting protein aggregation. Zwitterionic detergents uniquely offer some intermediate class properties that are superior to the other three detergent types in some applications offering the low denaturating and net zero charge characteristics of non-ionic detergents. Zwitterionics also efficiently disrupt protein aggregations.

Preferably, the detergent is an alkaline salt of a saturated unbranched C6-C20 alkyl sulphate or carbonate or is an isoprenyl salt. From the alkaline salts, lithium, sodium and potassium salt, especially lithium salts, are preferred.

The saturated unbranched C6-C20 alkyl sulphate may be an n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-icosayl sulphate. The saturated unbranched C6-C20 alkyl carbonate may be an n-hexyl, n-heptyl, n-octyl, n-nonyl, n-decyl, n-undecyl, n-dodecyl, n-tridecyl, n-tetradecyl, n-pentadecyl, n-bexadecyl, n-heptadecyl, n-octadecyl, n-nonadecyl, n-icosayl carbonate.

In a further preferred embodiment of the invention alkaline salt is a lithium salt, preferably a lithium salt of n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-icosayl sulphate or a lithium salt of n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-icosayl carbonate.

The method of producing the isolated protein of the invention may further comprise removing the marker or tag after step b) or step c). The removal of the marker may, for example, be carried out by cleaving off the marker. The marker may be cleaved-off by a suitable enzyme which specifically cleaves proteins at specific a cleaving site. The cleaving site could be a protease cleaving site, which may be located in a spacer between the marker or tag and the LBD. Examples of proteases are chymotrypsin, trypsin, elastase and plasmid, the corresponding cleaving sites are known to a person skilled in the art. Since the molecule to be purified is a protein, specific proteases, especially proteases from viruses that normally are attack plants are preferred. Examples of suitable specific proteases are thrombin, Factor Xa, Igase, TEV-protease from tobacco etch virus, protease PreScission (human rhinovirus 3C protease), enterokinase or Kex2 TEV-protease and PreScission are especially preferred.

Preferably, the alkaline salt of a saturated unbranched C6-C20 alkyl sulphate or carbonate is an alkaline salt of a saturated unbranched C9-C15 alkyl sulphate, preferably an alkaline salt of dodecyl sulphate, more preferably lithium dodecyl sulphate (LDS). In accordance with this, the saturated unbranched C9-C15 alkyl sulphate may be a lithium, sodium or potassium salt of n-nonyl sulphate, n-decyl sulphate, n-undecyl sulphate, n-dodecyl sulphate, n-tridecyl sulphate, n-tetradecyl sulphate, or n-pentadecyl sulphate, particularly lithium n-nonyl sulphate, lithium n-decyl sulphate, lithium n-undecyl sulphate, lithium n-dodecyl sulphate, lithium n-tridecyl sulphate, lithium n-tetradecyl sulphate, or lithium n-pentadecyl sulphate, more particularly lithium dodecyl sulphate, sodium dodecyl sulphate, potassium dodecyl sulphate, in particular lithium dodecyl sulphate.

Alternatively, the detergent is an isoprenyl salt. The isoprenyl salt may be any salt of isoprene, such isoprenyl acetate, isoprenyl diphosphate, isoprenyl pyrophosphate. Alternatively, the detergent is a terpene consisting of 1-4 isoprenyl-units and a hydroxyl-group, or an alkaline or ammounium salt of the corresponding carbonate, sulphate, phosphate or pyrophosphate particularly geraniol, farnesol, geranylpyrophosphate, farnesylpyrophosphate or geranylgeranylpyrophosphate.

Furthermore, steps b) and/or c) may be performed in the presence of an agonist for the LBD. The agonist may be the naturally occurring agonist or a functionally active derivative thereof or it may be an agonist different from the naturally occurring agonist. Naturally occurring agonistic ligands that bind to and activate nuclear receptors include lipophilic substances such as endogenous hormones, vitamins A and B and xenobiotic endocrine disrupters. For example, thyroid hormone receptors are activated by binding of thyroid hormone, particularly, thyroxine (T₄). The naturally occurring ligands for retinoic acid receptors are all-trans retinoic acid and 9-cis retinoic acid. Ligands for peroxisome proliferator-activated receptors are free fatty acids and eicosanoids; PPARγ is activated by PGJ₂ (a prostaglandine) and PPARα is activated by leukotriene B₄. Liver X receptor α and β form heterodimers with the obligate partner RXR. The heterodimer can be activated also with an LXR agonist (e.g. oxysterols) or an RXR agonists (such as 9-cis retinoic acid). Oxysterols are the oxygenated derivatives of cholesterol, such as 22 (R)-hydroxy cholesterol, 24 (S)-hydroxysterol. 27-hydroxy sterol and cholestenoic acid. Other agonists may be vitamin D (vitamin D receptor), steroids (estrogen receptor, progesterone receptor, androgen receptor), cortisol (glucocorticoid receptor), aldosterone (mineral corticoid receptor) or fatty acids (hepatocyte nuclear factor 4). Not naturally occurring agonistic ligands include dexamethasone for glucocorticoid receptor or diethylstilbestrol (DES) for estrogen receptor. Suitable agonistic ligands for the members of the ROR family include, cholesterol and derivatives thereof, such as cholesterol sulfate, or melatonin.

As detailed above, the isolated protein is produced by culturing a cell comprising nucleic acid coding for the protein under suitable conditions conducive to the production of the protein. The cell may be selected from the group consisting of an animal cell, a plant cell, a yeast cell. Suitable cells of animal cells include mammalian cells, in particular human cells. Examples of those cells are mentioned above. Alternatively, the cell may be a yeast cell, such as an *E*. *coli* cell, or an insect cell.

An exemplary method of producing the isolated protein is described in Example 1.

The present invention uses an isolated protein comprising a ligand binding domain of a nuclear receptor in controllable form produced according to the method as disclosed above.

The skilled person will understand that the isolated protein can be as defined above in any of the embodiments of the invention, particularly of the preferred embodiments of the invention.

The present invention discloses the use of an isolated protein for the identification of a ligand for a ligand binding domain of a nuclear receptor, particularly of an agonist or antagonist, especially an agonist. In accordance therewith, the isolated protein may be used for the identification of a ligand for LBD of a nuclear receptor, particularly an agonist or an antagonist, e.g., by switching on or switching off the downstream signal transduction. In accordance with this, any downstream signal may be detected or evaluated in order to detect binding of a ligand. If it is searched for an agonist, the agonist may be identified by the activation of the downstream signal pathway. On the other hand, an antagonist might be identified by switching off the downstream signal transduction. In case of an antagonist, it might be necessary to use the combination of an agonist and a potential antagonist in order to detect the deactivation of agonist-induced signal transduction by the antagonist. As detailed above, the signal transduction of nuclear receptor in general includes formation of a multimer, particularly a dimer, binding of a co-activator and/or co-repressor, or alteration in gene transcription, often an induction of gene transcription, accordingly, production ofmRNA and a protein and, therefore, changed cell function.

In accordance with the above described signal transduction, changed signal transduction may be assessed at each level of signal transduction including binding of the protein of interest to a second protein (such as co-activators or co-repressors), binding to a target gene, determination of the amount of mRNA or a protein, or altered cell function. Methods of determining binding of a protein to a further protein, or a target gene are well known to the skilled person and include those defined herein. Methods for determining the amount of mRNA or protein are also well known to the skilled person. Methods of observing changed cell function largely depend on the type of cell function and are also well known to the skilled practitioner.

The present invention uses a test system comprising
- the isolated protein,
- a co-factor, and
- means for detecting the interaction between the protein and the co-factor upon binding of a ligand, especially an agonist, to the ligand binding domain of the nuclear receptor.

The isolated protein may be any of the proteins as specified in the above aspects and embodiments, particularly preferred embodiments. Additionally, the co-factor (also referred to as co-regulator, co-regulatory protein, or transcription co-regulator including also a co-activator or a co-repressor; see also above) is bound by a nuclear receptor activated by the binding of an agonistic ligand, whereas the co-repressor is bound by a nuclear receptor upon binding of an antagonistic ligand. A common feature of nuclear receptor co-activators is that they contain one or more LXXLL binding motifs (a continuous sequence of five amino acids, where L = leucine and X = any amino acid) referred to as NR (nuclear receptor) boxes. The LXXLL binding motifs have been shown to bind to a structure on the surface of the LBD of nuclear receptors. Examples include:
- NCOA-1 (nuclear receptor co-activator 1) / SRC-1 (steroid receptor co-activator-1)
- NCOA-2 (nuclear receptor co-activator 2) / GRIP-1 (glucocorticoid receptor interacting protein 1)
- NCOA-3 (nuclear receptor co-activator 3) / AIB-1 (amplified in breast)
- NCOA-4 (nuclear receptor co-activator 4) / ARA 70 (androgen receptor associated protein 70)
- NCOA-5 (nuclear receptor co- activator 5)
- NCOA-6 (nuclear receptor co-activator 6)
- NCOA-7 (nuclear receptor co-activator 7)
- PGC-1 (proliferator-activated receptor γ co-activator 1)
- CBP (cAMP responsive element-binding (CREB) protein-binding protein)
- PCAF (p300/CBP associating factor)
- ARA 54 (androgen receptor associated protein 54)
- ARA 55 (androgen receptor associated protein 55)

Co-repressor proteins also bind to the surface of the ligand binding domain of nuclear receptors, but through an LXXXIXXX (I/L) motif of amino acids (where L = leucine, I = isoleucine and X = any amino acid). Additionally, co-repressors preferably bind to the nuclear receptor in inactivated form, free of an agonist or, possibly, in antagonist-bound form. Examples of co-receptors include:
- NCOR-1 (nuclear receptor co-repressor)
- NCOR-2 (nuclear receptor co-repressor) / SMRT (silencing mediator (co-repressor) for retinoid and thyroid hormone receptors)
- LCoR (ligand-dependent co-repressor)
- RCOR (REST co-repressor)
- CtBP 602618
- Rb (retinoblastoma protein)
- SIN3 (SIN3a, SIN3b)

Co-factors with dual function activator/repressor include:
- PELP-1 (proline, glutamic acid and leucine-rich protein 1)
- NSD-1
- RIP-14 (RXR-interacting protein 14)

The co-activator may be chosen depending on the LBD which is encompassed in the isolated protein according to the invention. The skilled person will understand that the co-factor depends on the signal transduction of the nuclear receptor the LBD is derived from and he will be able to choose a suitable co-factor for the test system of the invention to detect the interaction between the co-factor and the isolated protein comprising the LBD upon binding of a ligand.

In a preferred embodiment of the invention, the co-factor is glucocorticoid receptor-inactivating protein-1 (GRIP-1) or steroid receptor co-activator-1 (SRC-1), optionally, labelled with a marked, preferably a tag.

GRIP-1 is a transcriptional co-regulatory protein which contains several nuclear receptor interacting domains and an intrinsic histone acetyl transferase activity. GRIP-1 is recruited to DNA promotion sites by ligand-activated nuclear receptors, such as ROR, particularly RORα. Grip-1, in turn, acetylates histones which promotes DNA transcription. GRIP-1 is also referred to as SRC-2 (steroid receptor co-activator-2) or transcriptional mediators / intermediary factor 2 (TIF-2) or nuclear receptor co-activator 2 (NCOA2).

SCR-1 is also a transcriptional co-regulatory protein which also contains several nuclear receptor interaction domains and an instrinsic histone acetyl transferase activity. SRC-1 is recruited to DNA promotion sites by ligand-activated nuclear receptors and, in turn, acetylates histones, which promotes downstream DNA transcription. SRC-1 is also referred to as nuclear receptor co-activator-1 (NCOA-1).

In one embodiment of the invention, the co-factor may be labelled with a marker, preferably with a tag. The marker or tag may be defined as above. The marker may be used for purification of the co-factor or it may be used in order to detect the interaction between the isolated protein and the co-factor. Suitable markers include antibodies, antigens, enzymes, radiolabels, etc. However, it should be understood that the co-factor should be labelled in a manner still allowing interaction with other components of the test system in order to allow detection of a signal indicative of the interaction between the isolated protein and the co-factor.

In a preferred embodiment of the invention, the test system is designed in a manner, wherein the proximity of the protein to the co-factor induces a detectably signal.
The proximity of the isolated protein and the co-factor may be reached by binding of the isolated protein to the co-factor. The induction of a signal may be effected by labeling of each of the components, wherein the proximity of the labels induces a detectable signal. The induced detectable signal may be a chemiluminescent signal, a change in color, a fluorescence signal, a radiation or any other suitable signal. The signal may be induced by interaction of two labels, wherein each label is bound to one component of the test system, i.e. the isolated protein and the co-factor. Such signals include a radiolabel, such as ¹²⁵I, one the one hand and a suitable quencher on the other hand in order to detect proximity in a scintillation counter (scintillation proximity assay). The components may encompass antigens accessible to antibodies labeled in a manner to detect FRET (fluorescence resonance energy transfer, see below). In another alternative, one of the proteins has bound to its surface a biomolecule capable of phosphorylation by a kinase and the other component has a trivalent metal ion complexed to its surface, e.g., via a suitable linker such as nitrilotriacetic acid, iminodiacetic acid or an appropriately substituted N-containing heterocycle, for example a triazoheterocycle, for example a triazocyclononaneononane, such as 1-propylamino-4-acetato-1,4,7-triazacyclononane. A chemiluminescent signal is generated when the donor and acceptor particles are in close proximity, which occurs upon binding of the protein to the co-factor (luminescent proximity assay).

In one preferred embodiment of the invention, the means for detecting the interaction between the protein and the co-factor include at least one antibody specific for the protein or the co-factor. As detailed above, the isolated protein may include an antigen for a specific antibody and the detection of the antibody. In an even more preferred embodiment of the invention, the test system comprises two antibodies, wherein the first antibody is specific for the protein and the second antibody is specific for the co-factor. The antibody may be labeled with a suitable marker, which is indicative of the presence of the respective component. Alternatively, the above-mentioned primary antibody may be detected by a suitable secondary antibody directed against the primary antibody. The secondary antibody may be used in order to detected the presence of the primary antibody, e.g. when bound to the secondary antibody. The primary or secondary antibody may be labeled with a marker as defined above, for example, an enzyme, a radiolabel, a fluorescence marker, a chemiluminescent marker, etc. Alternatively, the components (the protein and/or the co-factor) may encompass a tag, which is detectable with a suitable antibody.

The test system may be used in a manner that a purification system for the separation of at least one component of the complex is used and the presence of the complex or of each of the components of the complex of the protein and the co-factor is detected. For example, the complex may be purified by gel electrophoresis, column chromatography, affinity purification, etc. and the complex may be detected by the presence of one signal or two signals indicative of one component of the complex or both components of the complex. For example, if a separation technique is used which is specific for one complex of the component, the detection method may be limited to the other component. Alternatively, the purification method may be not specific for one of the components, such as gel electrophoresis, and the formation of the complex may be detected by two signals, e.g. two antibodies labeled with distinguishable fluorescence markers, wherein the presence of both fluorescence markers e.g. at the same area of a gel is indicative of the complex.

In a still further embodiment of the invention, the test system of the invention is characterized in that
a) the first antibody is labeled with a donor moiety for fluorescence resonance energy transfer (FRET) and the second antibody is labeled with an acceptor moiety for FRET or vice versa; or
b) the first antibody is labeled with a donor moiety for time-resolved fluorescence resonance energy transfer (TR-FRET) and the second antibody is labeled with an acceptor moiety for TR-FRET or vice versa; or
c) the first antibody is labeled with a donor moiety for Amplified Luminescence Proximity Homogeneous Assay (ALPHA) and the second antibody is labeled with an acceptor moiety for ALPHA or vice versa.

Fluorescence resonance energy transfer (FRET) describes a radiation-free energy transfer between two chromophores. A donor chromophore in its excited state can transfer energy by a non-radiative long-range dipole-dipole coupling mechanism to an acceptor fluorophore in close proximity (typically <10 nm). As both molecules are fluorescent, the energy transfer is often referred to as "fluorescence resonance energy transfer", although the energy is not actually transferred by fluorescence. FRET is a useful tool to detect and quantify protein-protein interactions, protein-DNA interactions, and protein-conformational changes. For monitoring binding of one protein to another or one protein to DNA, one of the molecules is labeled with a donor and the other with an acceptor and these fluorophore-labeled molecules are mixed. When they are present in an unbound state, donor emission is detected upon donor excitation. Upon binding of the molecules, the donor and acceptor are brought in proximity and the acceptor emission is predominantly observed because of the intermolecular FRET from the donor to the acceptor. Suitable neighbors for FRET are known in the art and the skilled practitioner will be able to choose a suitable combination of labels for both antibodies. As used herein with respect to donor and corresponding acceptor, "corresponding" refers to an acceptor fluorescent moiety having an emission spectrum that overlaps with the excitation spectrum of the donor. However, both signals should be separable from each other. Accordingly, the wavelength maximum of the emission spectrum of the acceptor should preferably be at least 30 nm, more preferably at least 50 nm, such as at least 80 nm, at least 100 nm or at least 150 nm greater than the wavelength maximum of the excitation spectrum of the donor.

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC-Red 610, LC -Red 640, LC-Red 670, LC -Red 705, Cy5, Cy5,5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (e.g., Europium, or Terbium). Donor and acceptor fluorescent moieties can be obtained, for example, from Molecular Probes (Junction City, OR) or Sigma Chemical Co. (St. Louis, MO).

Alternatively, time-resolved fluorescence resonance energy transfer (TR-FRET) may be used for the test system used in the present invention. TR-FRET unites TRF (time-resolved fluorescence) and the FRET principle. This combination combines the low background benefits of TRF and the homogeneous assay format of FRET. While FRET has already been described above, TRF takes advantage of the unique properties of lanthanides or any other donor with long half-life. Suitable donors for TR-FRET include, amongst others, lanthanide chelates (cryptates) and some other metal ligand complexes, which can have fluorescent half-life in the micro- to millisecond time range and which, therefore, also allow the energy transfer to occur in micro-to millisecond measurements. Fluorescence lanthanide chelates have been used as energy donors in the late seventies. The commonly used lanthanides include samarium (Sm), europium (Eu), terbium (Tb) and dysprosium (Dy). Because of their specific photophysical and spectral properties, complexes of lanthanides are of major interest for fluorescence application in biology. Specifically, they have a large stroke's shift and extremely long emission half-lives (from microseconds to milliseconds) when compared to more traditional fluorophores.

Usually, organic chromophores are used as acceptors. These include allophycocyanin (APC). Suitable details on TR-FRET as well as acceptors are described in WO 98/15830.

In a further embodiment of the invention, the test system used in the invention is adapted for an amplified luminescence proximity homogeneous assay (ALPHA). ALPHA is a solution-based assay which was originally developed by Packard BioScience. ALPHA is a luminescence-based proximity assay, wherein one interaction partner is attached to donor beads, while the other is coupled to acceptor beads, both with a diameter of only about 250 nm. A photosensitizer compound is embedded into the donor bead. With this compound upon illumination with laser light at a wavelength of about 680 nm, ambient oxygen is converted into energy-rich, short-life singlet oxygen. When no acceptor bead is in proximity, the singlet oxygen decays without producing a signal. If donor and acceptor bead are brought together (ca. 250 nm) by the biological interaction of the attached biomolecules, the singlet oxygen released by the donor bead initiates a luminescence/fluorescence cascade in the nearby acceptor bead, leading to a highly amplified signal in the 520-620 nm range. The luminescence signal is detected in a suitable reader. For more details regarding ALPHA techniques, see Ullman et al., 1994, Proc. Natl. Acad. Sci., USA 91, 5426-5430.

An exemplary test system and its use are described in Example 2 and illustrated in Figure 1C.

Still a further aspect of the present invention relates to a method of screening a ligand for a ligand binding domain of a nuclear receptor comprising the steps of:
a) contacting a test system comprising
   - an isolated protein comprising a ligand binding domain of a nuclear receptor wherein the ligand binding domain is activatable upon binding of an agonist to the ligand binding domain
   - a co-factor werein the co-factor is bound by the nuclear receptor upon binding of a ligand, and
   - means for detecting the interaction between the protein and the co-factor upon binding of a ligand, especially an agonist, to the ligand binding domain of the nuclear receptor with a substance and
b) detecting a measurable signal upon binding of the substance to the ligand binding domain, thereby identifying the substance as a ligand for the ligand binding domain,
wherein the binding of the substance to the ligand binding domain induces the binding of the co-factor to the protein and the binding of the co-factor to the protein induces the measurable signal.

In accordance with the present invention, the test system may be specified as detailed in the present description of the invention, particularly as detailed in the preferred embodiments. The test system may be encompassed in a cell or it may be a cell-free system. A cell-free system is preferred. The test system may be contacted with a test substance under conditions suitable to detect a measurable signal. This includes a suitable temperature, chemical environment (buffers, pH-value, etc.) as well as a suitable concentration of the substance and an appropriate time of contact.

After or simultaneously with the contacting, a signal is observed wherein the detection of a signal is indicative of a ligand for the LBD. The signal may be any signal as detailed above in the context of the test system of the invention and the isolated protein of the invention and its use.

In a preferred embodiment of the method of the invention, the test system includes a first antibody labeled with a donor for FRET or TR-FRET and a second antibody labeled with an acceptor moiety for FRET or T-FRET or vice versa. Additionally, the presence of FRET is indicative of an agonist.

In a preferred embodiment of the invention, the screening method is used for screening for a medicament for preventing and/or treating a coronary artery disease (CAD), arteriosclerosis, dyslipidemia, a neurodegenerative disease, sleep disorder, a disease of circadian rhythmicity or osteoporosis.

The aforementioned diseases involve ROR, particularly RORα. Accordingly, it is assumed that an agonist or an antagonist of an ROR, particularly an RORα LBD, will have a beneficial influence on these diseases and may therefore be used in order to prevent or treat these diseases.

In the following, the present invention is illustrated by figures and examples which are not intended to limit the scope of the present invention.

### FIGURES

Figure 1A shows a schematic illustration of RORα1, indicating the various domain of this nuclear receptor.
Figure 1B shows various constructs of the invention, wherein the LBD of RORα1 is linked to a cleavage site (TEV or PreScission (PreSci)), a His-tag (His) and optionally Glutathione-S-transferase-tag (GST).
Figure 1C shows an exemplary illustration of an FRET assay according to the invention. In this assay, RORα is detected by a specific antibody bound to FRET donor Europium (Eu). The co-activator (CA) is biotin labeled. The biotin label is detecting by streptavidin, which encompasses the FRET acceptor allophycocyanin (APC) marker. If an agonist is bound to RORα, the NR is activated a CA binds to the NR. Accordingly, APC is brought into proximity of Eu, leading to a detecting FRET signal (left panel). In the presence of an antagonist, CA does not bind to RORα, accordingly no FRET signal is produced (right panel).

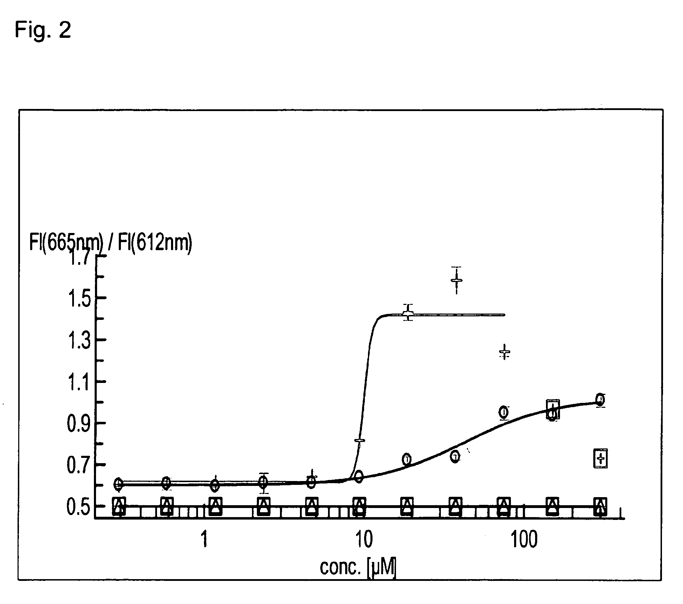

Figure 2 shows the results of the assay of Example 2. Cholesterol and cholesterol sulfate dose-dependently induce binding of a co-activator peptide SRC1-NR1+2 to RORα as measured by an increase in fluorescence intensity ratio in a fluorescence resonance energy transfer assay. The upper line (+) represents an assay perform in the presence of increasing concentrations of cholesterol sulfate, whereas the middle line (O) shows the assay carried out in the presence of increasing concentrations of cholesterol. The bottom lines (□ and Δ) represent experiments carried out in the presence of solvent only.

### EXAMPLES:

### Example 1: RORα- Expression, Purification and AD

Expression and purification of RORalpha protein was performed according to Kallen et al.. 2002, Structure, 10 (1697).

For expression and purification, DNA of RORα1 encompassing the ligand binding domain (LBD, amino acids 271-523) was cloned into the pVL1393 vector with an N-terminal stretch of 6 consecutive histidine-residues and a recognition sequence for the HRV 3C protease. Virus generation and expression in Sf9 insect cells was done following standard procedures. Infected cells were harvested 72 hrs post infection and cell pellets were stored at -80°C.

Frozen cell pellets were resuspended in 500 mM NaCl, 50 mM Tris, pH 8.0, 5 mM β-mercaptoethanol. Protease inhibitors (Complete EDTA-free, Roche Diagnostics) as well as Benzonase (Novagen) were added. The lysate was stirred for 30 minutes on ice and cells were finally disrupted by sonication. After centrifugation, the cleared lysate was loaded onto a Ni²⁺ - charged HisTrap FF (GE Healthcare) column equilibrated with 500 mM NaCl, 50 mM Tris, pH 8.0. Elution was done with a linear gradient over 30 CV to 500 mM NaCl, 500 mM imidazole, pH 8.0. Fractions were analyzed by SDS-PAGE and corresponding fractions were pooled and dialyzed against 30 mM Hepes, pH 7.0, 20 mM NaCl, 2 mM DTT, 5% glycerol.

For further purification, protein was loaded onto a HiTrap Q FF anion exchange column (GE Healthcare), equilibrated with 30 mM Hepes, pH 7.0, 20 mM NaCl, 2 mM DTT. Protein was eluted over a 20 CV gradient to 30 mM Hepes, pH 7.0, 1 M NaCl, 2 mM DTT. Corresponding fractions were pooled and further purified on a Superdex 200 26/60 gelfiltration column (GE Healthcare) equlilibrated in 150 mM NaCl, 5 mM DTT, 50 mM Tris, pH 7.5. Fractions containing RORαLBD were pooled and concentrated. The protein fractions were at least 95% pure as judged by SDS-PAGE and capillary electrophoresis (Agilent 2100 Bioanalyzer).

Protein (2.3 mg/ml) was aliquoted and stored at -80°C. Prior usage for *in vitro* assays, protein was dialyzed against 20 mM Tris, pH 7.5 for 8 hrs at room temperature. Lithium dodecylsulphate (LDS) was added to a final concentration of 3 mM and the mixture was further incubated at room temperature with gently shaking over night. Protein was aliqoted and stored at -80°C.

The purified and LDS-treated RORα LBD protein was shown to interact with glucocorticoid receptor-interacting protein 1 (GRIP1) and steroid receptor co-activator 1 (SRC1) in a co-factor recruitment fluorescence resonance energy transfer assay (FRET). Specifically the peptides GRIP1-NR1, GRIP1-NR2 and SRC1-NR1+2 - derived from the different nuclear receptor boxes (NR) of the two co-factors - were found to interact with RORα.

In all three cases cholersterol sulphate was able to induce co-factor binding dose-dependently with an EC₅₀ of 22 µM. 20 nM RORα LBD protein labeled with equimolar amounts of fluorescent anti-6xHis antibody were incubated with 400 nM of the respective biotin-labeled peptide and equimolar amounts of a streptavidin-tagged fluorophore in a buffer containing 50 mM Tris, 100 mM NaCl, 1 mM DTT and 0.1 % BSA for 2 hrs. Time resolved measurement of fluorescence ratios at 665 nm and 612 nm showed dose-dependent co-factor recruitment by known agonists like cholesterol sulphate and cholesterol.

### Example 2: TR-FRET assay measuring RORalphaLBD interaction with the LXXLL peptide, SRC1-NR1+2

### For the TR-FTET assay the following materials were used:

### Proteins:

- 6xHis-RORalpha:
   source : Protein Production (Lab. Thomas Langer) treated with 3 mM LDS, stock solution at 1.5 mg/ml, storage at -20°C, pipetting at room temperature, corresponding to 47,5 µM (with MW 31,6 kD), Concentration in the assay = 20 nM
- SRC1-NR1+2:
   source: JPT Peptide Technologies, stock solution at 250 µM, storage at -20°C,
   concentration in the assay = 400 nM

### Fluorophores/Labels:

- anti-6xHis antibody:
   source: Perkin Elmer # AD0110, stock solutions are at varying concentration with each batch, storage at -20°C, concentration in the assay = 20 nM
- strep-APC:
   source: Perkin Elmer # AD0201, stock solutions are at varying concentration with each batch, storage at +4°C after reconstitution, concentration in the assay = 200 nM (based on streptavidin)

### Agonist:

- Cholesterol sulfate:
   source: SIGMA #C9523, stock solution in DMSO (30 mM), storage at -20°C

### Buffer:

- 50 mM Tris, 100 mM NaCl, 1 mM DTT, 0,1 % Bovine Serum Albumin (add BSA fresh each day), adjust pH to 7,4

### Plates:

- Test plates: CORNING Costar # 3639 (96 well half area)

### The assay was carried out as follows:

All proteins, labels and compounds were diluted in the assay buffer just prior to assay setup. This assay was realised in 96 well half area plates. The final test volume was 20 µl. It is divided in 2 pipetting steps :

First, a mix containing 6xHis-RORalphaLBD protein, BiotinSRC1-NR1+2 peptide, anti-6xHis antibody and strep-APC was prepared on ice in the following concentrations:

| | | Test concentration (final): |
|---|---|---|
| 6xHis-RORalphaLBD | 27 nM | 20 nM |
| BiotinSRC1-NR1+2 | 533 nM | 400 nM |
| anti-6xHis antibody | 27 nM | 20 nM |
| strep-APC | 267 nM | 200 nM |

⇒ 15µl of this mix is prepipetted into a white 96 well plate

In a second step, 5 µl of the agonist compound was added as a 4-fold concentrated solution. Cholesterol sulphate was typically diluted from 300 µM final compound concentration downwards in 2-fold dilution steps and yields an EC₅₀ of about 10 µM. Plates were sealed to avoid evaporation.

The plates were incubated for 2h in subdued light at room temperature and read on a Tecan ULTRA at room temperature with 340 nm excitation and 612 nm as Europium reference and 665 nm as APC FRET signal. The results of this test are shown in Fig. 2.

## Claims

1. A method for screening for a ligand for a ligand binding domain of a nuclear receptor comprising the steps of:
a) contacting a test system comprising
- an isolated protein comprising a ligand binding domain of a nuclear receptor wherein the ligand binding domain is activatable upon binding of an agonist to the ligand binding domain
- a co-factor wherein the co-factor is bound by the nuclear receptor upon binding of a ligand, and
- means for detecting the interaction between the protein and the co-factor upon binding of a ligand, especially an agonist, to the ligand binding domain of the nuclear receptor
with a substance and
b) detecting a measurable signal upon binding of the substance to the ligand binding domain, thereby identifying the substance as a ligand for the ligand binding domain,
wherein the binding of the substance to the ligand binding domain induces the binding of the co-factor to the protein and the binding of the co-factor to the protein induces the measurable signal.

2. The method of claim 1, wherein the nuclear receptor is a Retinoic acid receptor-related Orphan Receptor (ROR).

3. The method of claim 2, wherein the nuclear receptor is RORα, RORβ or RORγ.

4. The method of any one of claims 1 to 3, wherein the protein further comprises a marker.

5. The method of claim 4, wherein the marker is a tag.

6. The method of claim 5, wherein the tag is selected from the group consisting of a His-tag, Arg-tag, Strep-tag, Flag-tag, T7-tag, V5-peptide-tag, c-Myc-tag, S-tag, HAT-tag, calmodulin-binding peptide-tag, chitin-binding peptide-tag, GST-tag and MBP-tag.

7. The method of any one of claims 4 to 6, wherein the marker or tag is removable from the protein by proteolytic cleavage at a specific cleavage site.

8. The method of any of claims 1 to 7 wherein the isolated protein comprises or consists of the sequence of SEQ ID NO: 1 or 2.

9. The method of any one of claims 1 to 8, wherein the co-factor is glucocorticoid receptor-inactivating protein- 1 (GRIP-I) or steroid receptor co-activator 1 (SRC1).

10. The method of claim 9, wherein the co-factor is labeled with a marker.

11. The method of any one of claims 1 to 10, wherein the means for detecting the interaction between the protein and the co-factor include at least one antibody specific for the protein or the co-factor.

12. The method of any one of claims 1 to 11, wherein the test system comprises two antibodies, wherein the first antibody is specific for the protein and the second antibody is specific for the co- factor.

13. The method of claim 12, wherein
(a) the first antibody is labeled with a donor moiety for fluorescence resonance energy transfer (FRET) and the second antibody is labeled with an acceptor moiety for FRET or vice versa; or
(b) the first antibody is labeled with a donor moiety for time-resolved fluorescence resonance energy transfer (TR-FRET) and the second antibody is labeled with an acceptor moiety for TR-FRET or vice versa; or
(c) the first antibody is labeled with a donor moiety for Amplified Luminescence Proximity Homogeneous Assay (ALPHA) and the second antibody is labeled with an acceptor moiety for ALPHA or vice versa.

14. The method of claim 13, wherein the presence of FRET is indicative for an agonist.

15. The method of any one of claims 1 to 14, wherein the method is used for screening for a medicament for preventing and/or treating a coronary artery disease (CAD), atherosclerosis, dyslipidemia, a neurodegenerative disease, sleep disorder, a disease of circadian rhythmicity or osteoporosis.

## Patentansprüche

1. Verfahren zur Suche nach einem Liganden für eine Ligandenbindungsdomäne eines nukleären Rezeptors, umfassend die Schritte:
a) Inkontaktbringen eines Testsystems, umfassend
- ein isoliertes Protein, das eine Ligandenbindungsdomäne eines nukleären Rezeptors umfasst, wobei die Ligandenbindungsdomäne bei Bindung eines Agonisten an die Ligandenbindungsdomäne aktivierbar ist,
- einen Cofaktor, wobei der Cofaktor bei Bindung eines Liganden von dem nukleären Rezeptor gebunden wird, und
- Mittel zum Nachweis der Wechselwirkung zwischen dem Protein und dem Cofaktor bei Bindung eines Liganden, vor allem eines Agonisten, an die Ligandenbindungsdomäne des nukleären Rezeptors,
mit einer Substanz und
b) Nachweisen eines messbaren Signals bei Bindung der Substanz an die Ligandenbindungsdomäne, wodurch die Substanz als ein Ligand für die Ligandenbindungsdomäne identifiziert wird, wobei die Bindung der Substanz an die Ligandenbindungsdomäne die Bindung des Cofaktors an das Protein und die Bindung des Cofaktors an das Protein das messbare Signal induziert.

2. Verfahren nach Anspruch 1, wobei es sich bei dem nukleären Rezeptor um einen ROR (Retinoic acid receptor-related Orphan Receptor) handelt.

3. Verfahren nach Anspruch 2, wobei es sich bei dem nukleären Rezeptor um RORα, RORβ oder RORγ handelt.

4. Verfahren nach einem der Ansprüche 1 bis 3, wobei das Protein ferner einen Marker umfasst.

5. Verfahren nach Anspruch 4, wobei es sich bei dem Marker um ein Tag handelt.

6. Verfahren nach Anspruch 5, wobei das Tag aus der Gruppe His-Tag, Arg-Tag, Strep-Tag, Flag-Tag, T7-Tag, V5-Peptid-Tag, c-Myc-Tag, S-Tag, HAT-Tag, Calmodulin-bindendes-Peptid-Tag, Chitin-bindendes-Peptid-Tag, GST-Tag und MBP-Tag ausgewählt ist.

7. Verfahren nach einem der Ansprüche 4 bis 6, wobei der Marker bzw. das Tag durch proteolytische Spaltung an einer spezifischen Spaltstelle von dem Protein abgetrennt werden kann.

8. Verfahren nach einem der Ansprüche 1 bis 7, wobei das isolierte Protein die Sequenz von SEQ ID NO: 1 oder 2 umfasst oder daraus besteht.

9. Verfahren nach einem der Ansprüche 1 bis 8, wobei es sich bei dem Cofaktor um Glucocorticoid-Rezeptor inaktivierendes Protein 1 (GRIP-I) oder Steroid-Rezeptor-Coaktivator 1 (SRC1) handelt.

10. Verfahren nach Anspruch 9, wobei der Cofaktor mit einem Marker markiert ist.

11. Verfahren nach einem der Ansprüche 1 bis 10, wobei die Mittel zum Nachweis der Wechselwirkung zwischen dem Protein und dem Cofaktor wenigstens einen für das Protein oder den Cofaktor spezifischen Antikörper enthalten.

12. Verfahren nach einem der Ansprüche 1 bis 11, wobei das Testsystem zwei Antikörper umfasst, wobei der erste Antikörper für das Protein und der zweite Antikörper für den Cofaktor spezifisch ist.

13. Verfahren nach Anspruch 12, wobei man
(a) den ersten Antikörper mit einer Donorgruppierung für Fluoreszenzresonanz-Energietransfer (FRET) und den zweiten Antikörper mit einer Akzeptorgruppierung für FRET oder umgekehrt markiert; oder
(b) den ersten Antikörper mit einer Donorgruppierung für zeitaufgelösten Fluoreszenzresonanz-Energietransfer (TR-FRET) und den zweiten Antikörper mit einer Akzeptorgruppierung für TR-FRET oder umgekehrt markiert; oder
(c) den ersten Antikörper mit einer Donorgruppierung für ALPHA (Amplified Luminescence Proximity Homogeneous Assay) und den zweiten Antikörper mit einer Akzeptorgruppierung für ALPHA oder umgekehrt markiert.

14. Verfahren nach Anspruch 13, wobei das Vorliegen von FRET auf einen Agonisten hindeutet.

15. Verfahren nach einem der Ansprüche 1 bis 14, wobei das Verfahren für die Suche nach einem Arzneimittel zur Vorbeugung und/oder Behandlung einer koronaren Herzkrankheit (KHK), von Atherosklerose, Dyslipidämie, einer neurodegenerativen Erkrankung, von Schlafstörung, einer Krankheit der circadianen Rhythmik oder von Osteoporose verwendet wird.

## Revendications

1. Procédé pour cribler un ligand pour un domaine de liaison de ligand d'un récepteur nucléaire comprenant les étapes de :
a) mise en contact d'un système de test comprenant
- une protéine isolée comprenant un domaine de liaison de ligand d'un récepteur nucléaire, le domaine de liaison de ligand étant activable par liaison d'un agoniste au domaine de liaison de ligand
- un cofacteur, le cofacteur étant lié par le récepteur nucléaire par liaison d'un ligand, et
- des moyens pour détecter l'interaction entre la protéine et le cofacteur par liaison d'un ligand, en particulier un agoniste, au domaine de liaison de ligand du récepteur nucléaire
avec une substance et
b) détection d'un signal mesurable par liaison de la substance au domaine de liaison de ligand, de manière à identifier la substance en tant que ligand pour le domaine de liaison de ligand,
la liaison de la substance au domaine de liaison de ligand induisant la liaison du cofacteur à la protéine et la liaison du cofacteur à la protéine induisant le signal mesurable.

2. Procédé de la revendication 1, le récepteur nucléaire étant un récepteur orphelin associé au récepteur d'acide rétinoïque (ROR).

3. Procédé de la revendication 2, le récepteur nucléaire étant RORα, RORβ ou RORγ.

4. Procédé de l'une quelconque des revendications 1 à 3, la protéine comprenant en outre un marqueur.

5. Procédé de la revendication 4, le marqueur étant une étiquette.

6. Procédé de la revendication 5, l'étiquette étant choisie dans le groupe constitué d'une étiquette His, une étiquette Arg, une étiquette Strep, une étiquette Flag, une étiquette T7, une étiquette de peptide V5, une étiquette c-Myc, une étiquette S, une étiquette HAT, une étiquette de peptide de liaison de la calmoduline, une étiquette de peptide de liaison de la chitine, une étiquette GST et une étiquette MBP.

7. Procédé de l'une quelconque des revendications 4 à 6, le marqueur ou l'étiquette étant séparable de la protéine par clivage protéolytique à un site de clivage spécifique.

8. Procédé de l'une quelconque des revendications 1 à 7, la protéine isolée comprenant ou étant constituée de la séquence de SEQ ID NO: 1 ou 2.

9. Procédé de l'une quelconque des revendications 1 à 8, le cofacteur étant la protéine inactivant le récepteur de glucocorticoïde 1 (GRIP-1) ou le co-activateur de récepteur de stéroïde 1 (SRC1).

10. Procédé de la revendication 9, le cofacteur étant marqué avec un marqueur.

11. Procédé de l'une quelconque des revendications 1 à 10, les moyens pour détecter l'interaction entre la protéine et le cofacteur comprennent au moins un anticorps spécifique pour la protéine ou le cofacteur.

12. Procédé de l'une quelconque des revendications 1 à 11, le système de test comprenant deux anticorps, le premier anticorps étant spécifique pour la protéine et le deuxième anticorps étant spécifique pour le cofacteur.

13. Procédé de la revendication 12, dans lequel
(a) le premier anticorps est marqué avec un fragment donneur pour transfert d'énergie par résonance en fluorescence (FRET) et le deuxième anticorps est marqué avec un fragment accepteur pour FRET ou réciproquement ; ou
(b) le premier anticorps est marqué avec un fragment donneur pour transfert d'énergie par résonance en fluorescence à résolution temporelle (TR-FRET) et le deuxième anticorps est marqué avec un fragment accepteur pour TR-FRET ou réciproquement ; ou
(c) le premier anticorps est marqué avec un fragment donneur pour essai homogène par luminescence de proximité amplifiée (ALPHA) et le deuxième anticorps est marqué avec un fragment accepteur pour ALPHA ou réciproquement.

14. Procédé de la revendication 13, la présence de FRET étant indicatrice d'un agoniste.

15. Procédé de l'une quelconque des revendications 1 à 14, le procédé étant utilisé pour cribler un médicament pour prévenir et/ou traiter une maladie coronarienne (CAD), l'athérosclérose, la dyslipidémie, une maladie neurodégénérative, un trouble du sommeil, une maladie du rythme circadien ou l'ostéoporose.
